# EUROPEAN PATENT APPLICATION

(11) **EP 4 488 379 A1**
(43) Date of publication of application: **08.01.2025**
(21) Application number: 23763562.8
(22) Date of filing: 02.03.2023
(51) Int. Cl.: C12Q 1/02

(54) **METHOD FOR DETECTING ENZYME ACTIVITY AND FLUORESCENT PROBE USED IN SAID METHOD**

(30) Priority: 02.03.2022 US 202263315778 P
(71) Applicant: The University of Tokyo, Bunkyo-ku, Tokyo 113-8654 (JP); RIKEN, Wako-shi, Saitama 351-0198 (JP)
(72) Inventor: URANO, Yasuteru, Tokyo 113-8654 (JP); KOMATSU, Toru, Tokyo 113-8654 (JP); SAKAMOTO, Shingo, Tokyo 113-8654 (JP); UKEGAWA, Tatsuya, Tokyo 113-8654 (JP); WATANABE, Rikiya, Wako-shi, Saitama 351-0198 (JP)
(74) Representative: ABG Intellectual Property Law, S.L.
(86) International application number: PCT/JP2023/007926
(87) International publication number: WO 2023/167305

(57) **Abstract**

An object of the present invention is to provide a method capable of detecting activity of an enzyme that strictly recognizes a substrate, such as acetylcholinesterase (AChE). There is provided a method for detecting activity of an enzyme in a biological sample using a microdevice, the method comprising a step of bringing the biological sample into contact with a substrate analog or a natural substrate, and a compound represented by General Formula (I) below or a salt thereof, wherein the substrate analog or the natural substrate generates a compound A having a thiol group (R-SH), a selenol group (R-SeH), or a poly sulfur atom (R-(S)n-H) (R is a hydrogen atom or an alkyl group, and n is 1 to 5) by a reaction with the enzyme, and fluorescence is observed or measured, the fluorescence being emitted or enhanced from a fluorescent product generated by the reaction of the compound represented by General Formula (I) or the salt thereof with the thiol group, the selenol group, or the poly sulfur atom.

## Description

### TECHNICAL FIELD

The present invention relates to a novel method for detecting enzyme activity using a fluorescent probe that selectively reacts with a thiol group, a selenol group, or the like generated by a reaction between an enzyme and a substrate analog in a microdevice, and a fluorescent probe used in the method.

### BACKGROUND ART

There are over thousands of enzymes in vivo, some of which have activity abnormalities associated with the development of various diseases. Knowing an abnormality of a specific enzyme activity in blood is also used in clinical situations as an index for determining the presence or absence of a disease.

However, currently, in a methodology for detecting an enzyme in blood, it is often difficult to detect an enzyme present in a significantly small amount in blood due to insufficient sensitivity, and in particular, in order to find an enzyme activity abnormality related to early diagnosis of a disease, there is a demand for high sensitivity of such an enzyme activity detection method.

On the other hand, the research group of the present inventors has hitherto performed profiling based on detection and activity of an enzyme in blood at a single molecule level using a group of microdevice-compatible enzyme activity detection fluorescent probes, and found new relevance to a disease.

The microdevice includes a microchamber type device, a liposome, a droplet, and the like. When the microchamber type device is described as an example, one device has hundreds of thousands of minute chambers, and a capacity of the device is as significantly small as about 10 to 100 fL. Therefore, the microdevice is designed so that sufficient sensitivity for detection can be obtained even with a fluorescent dye amount at a level generated by turning over from one molecule of enzyme.

Various experimental systems, in which activity of an enzyme can be evaluated at a single molecule level by taking full advantage of the properties of a highly sensitive fluorescence method using the microdevice, have been reported. As an example, in recent years, it has been reported by the present inventors that one molecule of an enzyme in blood is detected using a single molecule measurement method, such that it is possible to find activity of an enzyme present in blood at a low concentration that is difficult to detect by conventional protein analysis techniques, and expectations for pathological diagnosis by enzyme detection in blood have been focused (Non Patent Document 1).

Conventionally, as a detection system for enzyme activity, a fluorescent substrate analog whose fluorescence changes by a metabolic reaction is mainly used. For example, by using a fluorescent probe having a phosphoric acid ester structure as a reaction point, activity of enzymes such as an alkaline phosphatase and a protein tyrosine phosphatase that hydrolyze phosphoric acid ester has been detected (Non Patent Documents 1 and 2).

However, there are limited types of enzymes that can be detected using such a fluorescent substrate analog, and in particular, it is difficult to develop such a probe for an enzyme/substrate pair that has strict substrate recognition and is less likely to allow binding of a fluorophore to a substrate because of the need to bind a fluorescent substance having a relatively large molecular weight to a substrate.

Examples of such an enzyme include acetylcholinesterase (AChE), and AChE is an esterase present in the brain and muscle and highly selectively hydrolyzes acetylcholine. Since the enzyme strictly recognizes a choline structure, it is known that development of a conventional fluorescent probe is difficult.

### Reference List

### Non Patent Documents

Non Patent Document 1: Sakamoto, S. et al. Multiplexed single-molecule enzyme activity analysis for counting disease-related proteins in biological samples. Sci. Adv vol. 6 https://www.science.org (2020).
Non Patent Document 2: Obayashi, Y, Iino, R. & Noji, H. A single-molecule digital enzyme assay using alkaline phosphatase with a cumarin-based fluorogenic substrate. Analyst 140, 5065-5073 (2015).

### SUMMARY OF THE INVENTION

### Technical Problem

An object of the present invention is to provide a method capable of detecting activity of an enzyme such as acetylcholinesterase (AChE) that strictly recognizes a substrate by a high sensitivity assay at a single molecular level using a microdevice. In addition, another object of the present invention is to provide a fluorescent probe that can be used for such a method.

### Solution to Problem

As a result of intensive studies to solve the above problems, the present inventors have focused on a coupled assay system that selectively detects a specific molecule generated by a metabolic reaction of a non-fluorescent natural substrate (or substrate analog). Then, using this methodology, the present inventors have found that it is possible to detect activity of an enzyme having high substrate specificity such as acetylcholinesterase by generating a thiol group, a selenol group, or the like by a reaction between the enzyme and a substrate analog in a microdevice, and using a fluorescent probe that selectively reacts with these groups, thereby completing the present invention.

That is, the present invention has the following features.
[1] A method for detecting activity of an enzyme in a biological sample using a microdevice, the method comprising a step of bringing the biological sample into contact with a substrate analog or a natural substrate, and a compound represented by General Formula (I) below or a salt thereof,
   wherein the substrate analog or the natural substrate generates a compound A having a thiol group (R-SH), a selenol group (R-SeH), or a poly sulfur atom (R-(S)n-H) (R is a hydrogen atom or an alkyl group, and n is 1 to 5) by a reaction with the enzyme, and
   fluorescence is observed or measured, the fluorescence being emitted or enhanced from a fluorescent product generated by the reaction of the compound represented by General Formula (I) or the salt thereof with the thiol group, the selenol group, or the poly sulfur atom, 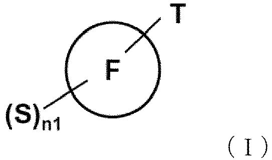
   wherein
      T is a reactive site that reacts with the thiol group (R-SH), the selenol group (R-SeH), or the poly sulfur atom (R-(S)n-H);
      S is a hydrophilic group selected from the group consisting of a sulfonic acid group, a carboxyl group, and a phosphonic acid group;
      n1 represents an integer of 1 to 10; 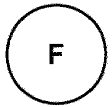
      represents a fluorophore, in which molecular design is carried out so that optical properties of the entire molecule of the compound change by the reaction of the compound represented by General Formula (I) or the salt thereof with the thiol group, the selenol group, or the poly sulfur atom; and
      S and T may be bonded to the fluorophore through a linker.
[2] The method according to [1], wherein the substrate analog has a thioester- or selenoester-like structure and generates a corresponding thiol group or selenol group by an enzyme reaction.
[3] The method according to [1] or [2], wherein the fluorophore is selected from the group consisting of a BODIPY-based fluorophore, a rhodamine-based fluorophore, a fluorescein-based fluorophore, a rhodol analog fluorophore, a cyanine-based fluorophore, a resorufin-based fluorophore, and a coumarin-based fluorophore.
[4] The method according to any one of [1] to [3], wherein the reactive site is selected from the group consisting of a nitroolefin group that may have a substituent, maleimides, an electron-deficient aryl group, and an alkyl or arylsulfonic acid group (R'-SO₃-; R' represents an alkyl group that may have a substituent or an aryl group that may have a substituent).
[5] The method according to [2], wherein the substrate analog is one or more selected from the group consisting of acetylthiocholine, butylthiocholine, or a sulfur-containing lipid.
[6] The method according to any one of [1] to [5], wherein the enzyme to be detected is one or more selected from the group consisting of esterase, lipase, glyoxalase, SAH hydrolase, N-acetyltransferase, cystathionine γ-lyase, S-adenosylhomocysteine hydrolase, and cystathionine β-synthase.
[7] The method according to [6], wherein the enzyme is acetylcholinesterase, phospholipase A2, or glyoxalase 2.
[8] A fluorescent probe for use in a method for detecting activity of an enzyme in a biological sample using a microdevice, the method comprising a step of bringing the biological sample into contact with a substrate analog or a natural substrate, and a compound represented by General Formula (I) below or a salt thereof,
   wherein the substrate analog or the natural substrate generates a compound A having a thiol group (R-SH), a selenol group (R-SeH), or a poly sulfur atom (R-(S)n-H) (R is a hydrogen atom or an alkyl group, and n is 1 to 5) by a reaction with the enzyme,
   fluorescence is observed or measured, the fluorescence being emitted or enhanced from a fluorescent product generated by the reaction of the compound represented by General Formula (I) or the salt thereof with the thiol group, the selenol group, or the poly sulfur atom, and
   the substrate analog generates a compound A having a thiol group, a selenol group (R-SeH), or a poly sulfur atom (R-(S)n-H) by a reaction with the enzyme,
   the fluorescent probe comprising a compound represented by General Formula (I) or a salt thereof, 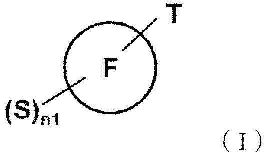
   wherein
      T is a reactive site that reacts with the thiol group (R-SH), the selenol group (R-SeH), or the poly sulfur atom (R-(S)n-H);
      S is a hydrophilic group selected from the group consisting of a sulfonic acid group, a carboxyl group, and a phosphonic acid group;
      n1 represents an integer of 1 to 10; 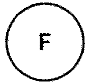
      represents a fluorophore, in which molecular design is carried out so that optical properties of the entire molecule of the compound change by the reaction of the compound represented by General Formula (I) or the salt thereof with the thiol group, the selenol group, or the poly sulfur atom; and
      S and T may be bonded to the fluorophore through a linker.
[9] The fluorescent probe according to [8], wherein the compound represented by General Formula (I) or the salt thereof is a compound represented by General Formula (II) below or a salt thereof, the compound having one to five hydrophilic groups selected from the group consisting of a sulfonic acid group, a carboxyl group, and a phosphonic acid group, and having one to three reactive sites that react with the thiol group, the selenol group, or the poly sulfur atom, 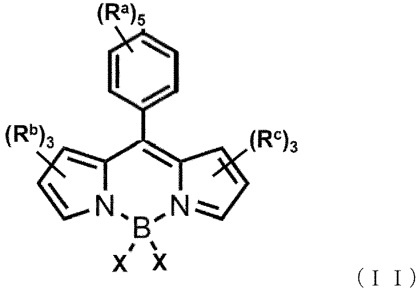 wherein
   R^{a} is each independently a hydrogen atom, a monovalent substituent, the hydrophilic group, an alkyl group having the hydrophilic group and having 1 to 6 carbon atoms, or the reactive site;
   R^{b} is each independently a hydrogen atom, a monovalent substituent, the hydrophilic group, an alkyl group having the hydrophilic group and having 1 to 6 carbon atoms, or the reactive site;
   R^{c} is each independently a hydrogen atom, a monovalent substituent, the hydrophilic group, an alkyl group having the hydrophilic group and having 1 to 6 carbon atoms, or the reactive site; and
   X is each independently selected from a fluorine atom, an alkyl group, an alkoxy group, an aryl group, the hydrophilic group, or an alkyl group having the hydrophilic group and having 1 to 6 carbon atoms.
[10] The fluorescent probe according to [9], wherein the compound represented by General Formula (II) or the salt thereof is a compound represented by General Formula (IIa) below or a salt thereof, 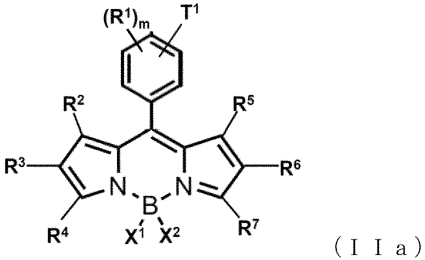 wherein
   R¹, when present, represents the same or different monovalent substituent present on a benzene ring, and the substituent may have the hydrophilic group;
   m is an integer of 0 to 4, and when m is 2 or more, each R¹ may be the same or different;
   T¹ is a reactive site that reacts with the thiol group, the selenol group, or the poly sulfur atom, and two or more T's may be bonded to the benzene ring;
   R² to R⁷ each independently represent a hydrogen atom, an alkyl group having 1 to 6 carbon atoms, the hydrophilic group, or an alkyl group having the hydrophilic group and having 1 to 6 carbon atoms; and
   X¹ and X² are each independently selected from a fluorine atom, an alkyl group, an alkoxy group, an aryl group, the hydrophilic group, or an alkyl group having the hydrophilic group and having 1 to 6 carbon atoms.
[11] The fluorescent probe according to [10], wherein the compound represented by General Formula (IIa) or the salt thereof is a compound represented by General Formula (IIb) below or a salt thereof, 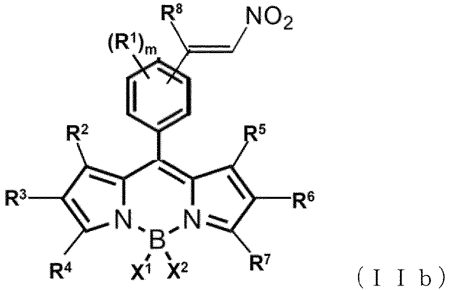 wherein
   R¹ to R⁷, X¹, X², and m are as defined in General Formula (IIa); and
   R⁸ represents a hydrogen atom or a substituted or unsubstituted alkyl group having 1 to 10 carbon atoms, and
   two or more nitroolefin moieties that may have a substituent may be bonded to the benzene ring,
   where at least one of R³ and R⁶ is the hydrophilic group.
[12] The fluorescent probe according to [10], wherein the compound represented by General Formula (IIa) or the salt thereof is a compound represented by General Formula (IIc) below or a salt thereof, 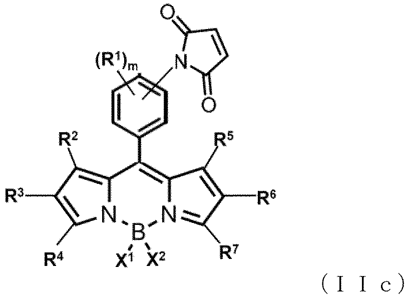 wherein
   R¹ to R⁷, X¹, X², and m are as defined in General Formula (IIa); and
   two or more maleimide groups may be bonded to the benzene ring,
   where at least one of R³ and R⁶ is the hydrophilic group.
[13] The fluorescent probe according to [8], wherein the compound represented by General Formula (I) or the salt thereof is a compound represented by General Formula (III) below or a salt thereof, the compound having one to five hydrophilic groups selected from the group consisting of a sulfonic acid group, a carboxyl group, and a phosphonic acid group, and having one to three reactive sites that react with the thiol group, the selenol group, or the poly sulfur atom, 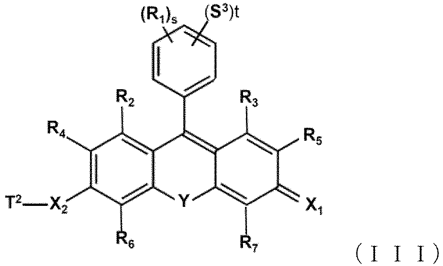 wherein
   R₁, when present, represents the same or different monovalent substituent present on a benzene ring;
   s is an integer of 0 to 4, and when s is 2 or more, each R₁ may be the same or different;
   S³ is a hydrophilic group selected from a sulfonic acid group, a carboxyl group, or a phosphonic acid group;
   t is 0 to 5;
   R₂ to R₇ each independently represent a hydrogen atom, an alkyl group having 1 to 6 carbon atoms, a halogen atom, the hydrophilic group, or an alkyl group having the hydrophilic group and having 1 to 6 carbon atoms;
   T² is a reactive site that reacts with the thiol group, the selenol group, or the poly sulfur atom;
   X¹ and X² are selected from combinations of (O,O), (O,NRs), (NR₉R₁₀,NR₈), and NR₉R₁₀,NR₈), where R₈ to R₁₀ are each independently a hydrogen atom, a substituted or unsubstituted alkyl group having 1 to 6 carbon atoms, a substituted or unsubstituted alkenyl group having 1 to 6 carbon atoms, an alkyl group having the hydrophilic group and having 1 to 6 carbon atoms, or an alkenyl group having the hydrophilic group and having 1 to 6 carbon atoms; and
   Y is selected from an oxygen atom, Si(Rₐ)(R_{b}), C(Rₐ)(R_{b}), P(=O)R_{c}, S(R_{d})₂, Ge(Rₐ)(R_{b}), a tellurium atom, or a bismuth atom,
   where Rₐ and R_{b} are each independently a substituted or unsubstituted alkyl group having 1 to 6 carbon atoms or a substituted or unsubstituted aryl group,
   R_{c} is a substituted or unsubstituted alkyl group having 1 to 6 carbon atoms or a substituted or unsubstituted phenyl group,
   R_{d} is the same or different substituted or unsubstituted aryl group, and
   the alkyl group, the aryl group, and the phenyl group of Rₐ to R_{d} may be substituted with the hydrophilic group.
[14] The fluorescent probe according to [13], wherein the reactive site of T² is any of the following: 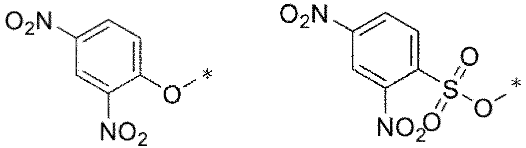 wherein * represents a binding site.
[15] The fluorescent probe according to any one of [8] to [14], wherein the substrate analog has a thioester- or selenoester-like structure and generates a corresponding thiol group or selenol group by an enzyme reaction.
[16] The fluorescent probe according to [15], wherein the substrate analog is one or more selected from the group consisting of acetylthiocholine, butylthiocholine, and a sulfur-containing lipid derivative.
[17] The fluorescent probe according to any one of [8] to [16], wherein the enzyme to be detected is one or more selected from the group consisting of esterase, lipase, glyoxalase, SAH hydrolase, N-acetyltransferase, cystathionine γ-lyase, S-adenosylhomocysteine hydrolase, and cystathionine β-synthase.
[18] The fluorescent probe according to [17], wherein the enzyme is acetylcholinesterase, phospholipase A2, or glyoxalase 2.
[19] The fluorescent probe according to any one of [8] to [18], wherein the fluorescent probe is used to detect activity of acetylcholinesterase in a biological sample including blood.
[20] A compound represented by General Formula (IIb) below or a salt thereof, 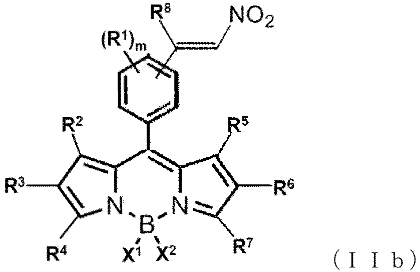 wherein
   R¹, when present, represents the same or different monovalent substituent present on a benzene ring, and the substituent may have a hydrophilic group selected from the group consisting of a sulfonic acid group, a carboxyl group, and a phosphonic acid group;
   m is an integer of 0 to 4, and when m is 2 or more, each R¹ may be the same or different;
   R² to R⁷ each independently represent a hydrogen atom, an alkyl group having 1 to 6 carbon atoms, the hydrophilic group, or an alkyl group having the hydrophilic group and having 1 to 6 carbon atoms;
   R⁸ represents a hydrogen atom or a substituted or unsubstituted alkyl group having 1 to 10 carbon atoms,
   provided that at least one of R³ and R⁶ is a sulfonic acid group, a carboxyl group, or a phosphonic acid group; and
   X¹ and X² are each independently selected from a fluorine atom, an alkyl group, an alkoxy group, an aryl group, the hydrophilic group, or an alkyl group having the hydrophilic group and having 1 to 6 carbon atoms.
[21] The compound or a salt thereof according to [20], wherein at least one of R³ and R⁶ is a sulfonic acid group.
[22] The compound or a salt thereof according to [20], wherein at least one of R³ and R⁶ is a carboxyl group.
[23] The compound or a salt thereof according to [20], wherein at least one of R³ and R⁶ is a phosphonic acid group.
[24] The compound or a salt thereof according to [20], wherein m is 1 to 2, and R¹ is an alkoxyl group having 1 to 6 carbon atoms.
[25] The compound or a salt thereof according to [20], wherein a nitroolefin moiety is attached to the 2- or 4-position of the benzene ring.
[26] The compound or a salt thereof according to [22], wherein R³ to R⁶ are all methyl groups.
[27] A compound selected from the following or a salt thereof:

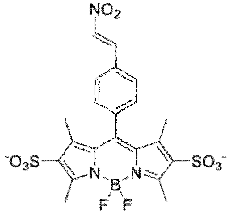

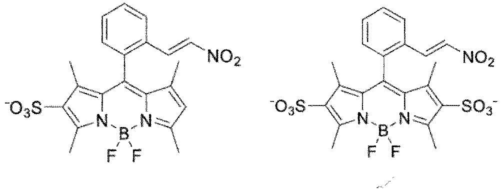

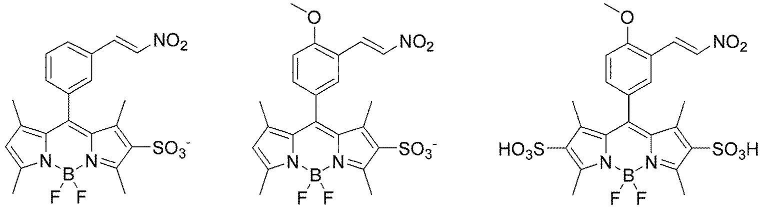

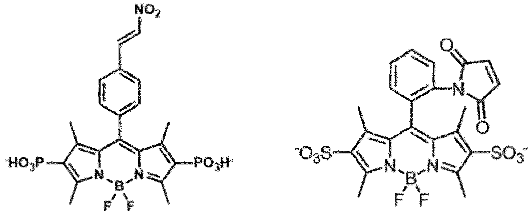

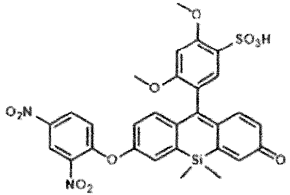

### Effects of the Invention

According to the present invention, it is also possible to effectively detect enzyme activity of an enzyme that strictly recognizes a substrate, such as acetylcholinesterase (AChE), phospholipase A2, or glyoxalase 2.

In addition, the detection method of the present invention is performed using a microdevice, such that it is possible to perform single molecule enzyme activity analysis of these enzymes with high sensitivity that reflects individuality (based on post-translational modification, difference in protein-protein interactions, and the like) of each molecule.

### BRIEF DESCRIPTION OF THE DRAWINGS

Fig. 1 illustrates a conceptual diagram of a detection method of the present invention using a non-limiting example of a combination of a substrate analog, an enzyme, and a compound represented by General Formula (I).
Fig. 2 illustrates a reaction formula showing how a non-limiting combination of a substrate analog that can be used in the detection method of the present invention and an enzyme generates an SH group.
Fig. 3 illustrates a polychromatic assay in which AChE is detected using an acetyl thioester as a substrate analog and a compound of General Formula (IIa) as a fluorescent probe, and BChE is detected using an acetyl selenoester as a substrate analog and a compound of General Formula (III) as a fluorescent probe.
Fig. 4 illustrates optical properties of a compound 1, and a compound 2 obtained in Synthesis Example 2.
Fig. 5 illustrates the results of examination of microdevice compatibility of the compounds 1 and 2.
Fig. 6 illustrates optical properties of a compound 4 and a compound 9.
Fig. 7 illustrates the results of examination of microdevice compatibility of compounds 4 and 9.
Fig. 8 illustrates the results of examining optical properties of a compound 17.
Fig. 9 illustrates the results of examination of microdevice compatibility of the compound 17.
Fig. 10 illustrates the results of analyzing a mixture of five compounds of the present invention by LC-MS.
Fig. 11 illustrates the results of the microdevice compatibility of the compounds 1, 2, 4, 9, and 17.
Fig. 12 illustrates a schematic diagram of detection of an enzyme in blood using a microdevice.
Fig. 13 illustrates the results of a test for detecting an enzyme in blood using the compound 17.
Fig. 14 illustrates the results of time course analysis of a microdevice containing human plasma in Example 4.
Fig. 15 illustrates the results of activity pattern confirmation in Example 4.
Fig. 16 illustrates the results of a detection experiment of Glyoxalase 2 (GLO2).
Fig. 17 illustrates the results of detecting activity of acetylcholinesterase in blood by using a probe of the present invention having a nitrophenol structure.
Fig. 18 illustrates the activity detection results of AChE and phospholipase (PLA2) using a probe having a maleimide structure.

### DETAILED DESCRIPTION OF THE INVENTION

In the present specification, "halogen atom" means a fluorine atom, a chlorine atom, a bromine atom, or an iodine atom.

In the present specification, "alkyl" may be any aliphatic hydrocarbon group that is linear, branched, cyclic, or in a combination thereof. The number of carbon atoms in the alkyl group is not particularly limited, but is, for example, 1 to 6 (C₁₋₆), 1 to 10 (C₁₋₁₀), 1 to 15 (C₁₋₁₅), or 1 to 20 (C₁₋₂₀). When the number of carbon atoms is specified, it means an "alkyl" having the number of carbon atoms within that numerical range. For example, C₁₋₈ alkyls include methyl, ethyl, n-propyl, isopropyl, n-butyl, isobutyl, sec-butyl, tert-butyl, n-pentyl, isopentyl, neo-pentyl, n-hexyl, isohexyl, n-heptyl, n-octyl, and the like. In the present specification, an alkyl group may have one or more optional substituents. Examples of such substituents include, but are not limited to, an alkoxy group, a halogen atom, an amino group, a mono- or di-substituted amino group, a substituted silyl group, and acyl. When an alkyl group has two or more substituents, they may be the same as or different from each other. The same is also true for the alkyl moiety of other substituents (for example, an alkoxy group, an arylalkyl group, or the like) including an alkyl moiety.

In the present specification, when certain functional groups are defined as "optionally substituted", the type of substituent, substitution position, and the number of substituents are not particularly limited, and when there are two or more substituents, they may be the same as or different from each other. Examples of the substituents include, but are not limited to, an alkyl group, an alkoxy group, a hydroxyl group, a carboxyl group, a halogen atom, a sulfo group, an amino group, an alkoxycarbonyl group, and an oxo group. Other substituents may be present in these substituents. Examples of such cases include, but are not limited to, an alkyl halide group and a dialkylamino group.

In the present specification, "alkoxy group" is a structure in which the above alkyl group is bonded to an oxygen atom, and examples thereof include saturated alkoxy group that is linear, branched, cyclic, or in a combination thereof. Preferred examples thereof include a methoxy group, an ethoxy group, an n-propoxy group, an isopropoxy group, a cyclopropoxy group, an n-butoxy group, an isobutoxy group, a s-butoxy group, a t-butoxy group, a cyclobutoxy group, a cyclopropylmethoxy group, an n-pentyloxy group, a cyclopentyloxy group, a cyclopropylethyloxy group, a cyclobutylmethyloxy group, an n-hexyloxy group, a cyclohexyloxy group, a cyclopropylpropyloxy group, a cyclobutylethyloxy group, and a cyclopentylmethyloxy group.

### 1. Method for Detecting Enzyme Activity

One embodiment of the present invention is a method for detecting activity of an enzyme in a biological sample using a microchamber type device, the method (hereinafter, also referred to as a "detection method of the present invention) comprising a step of bringing the biological sample into contact with a substrate analog or a natural substrate, and a compound represented by General Formula (I) below or a salt thereof,
wherein the substrate analog or the natural substrate generates a compound A having a thiol group (R-SH), a selenol group (R-SeH), or a poly sulfur atom (R-(S)n-H) (R is a hydrogen atom or an alkyl group, and n is 1 to 5) by a reaction with the enzyme, and
fluorescence is observed or measured, the fluorescence being emitted or enhanced from a fluorescent product generated by the reaction of the compound represented by General Formula (I) or the salt thereof with the thiol group, the selenol group, or the poly sulfur atom.

Without intending to be bound by theory, the detection method of the present invention uses a coupled assay methodology using a thiol, selenol, or the like. That is, it is possible to detect activity of an enzyme having high substrate specificity such as acetylcholinesterase by generating a thiol group, a selenol group, or the like by a reaction between an enzyme to be detected and a substrate analog, and using a fluorescent probe that selectively reacts with these groups.

Although there has been a coupled assay using a thiol so far, the coupled assay is based on the conventional biochemical analysis methodology and handles 10⁸ to 10⁹ or more molecules as a group, but in the detection method of the present invention, analysis can be performed in consideration of a difference in activity for each molecule by using an enzyme activity detection method for each molecule, and such an object can be achieved particularly by an assay using a thiol.

As described above, with the detection method using the microdevice of the present invention, it is possible to detect activity of an enzyme having high substrate specificity such as acetylcholinesterase, and preferably, it is possible to detect activity of an enzyme having high substrate specificity at a single molecule level.

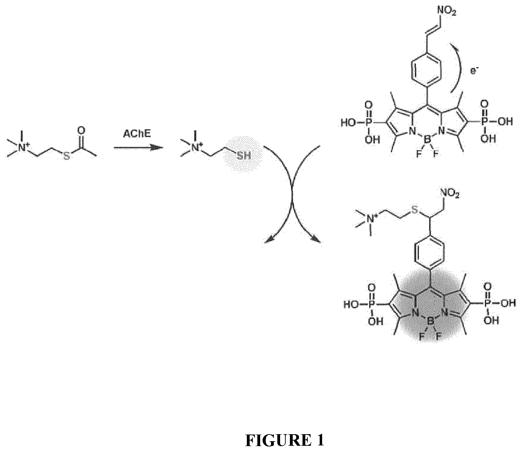

A conceptual diagram of the detection method of the present invention is illustrated in Fig. 1 using a non-limiting example.

Fig. 1 illustrates a coupled assay system in which, as a substrate analog, a compound having an SH group is generated by substitution of oxygen of an ester structure of choline with sulfur (S) and hydrolysis of a thioester by acetylcholinesterase (AChE), and the compound is selectively detected by a fluorescent probe having a reactive site with the SH group. Thus, acetylcholinesterase can be detected with high sensitivity.

The substrate analog used in the detection method of the present invention reacts with an enzyme to generate a compound A having a thiol group (R-SH), a selenol group (R-SeH), or a poly sulfur atom (R-(S) n-H). Here, R is a hydrogen atom or an alkyl group. n is an integer of 1 to 5.

The substrate analog preferably has a structure in which one of the oxygen atoms of the carboxylic acid ester of the substrate molecule of the enzyme is substituted with sulfur or selenium.

The substrate analog preferably has a thioester- or selenoester-like structure and generates a corresponding thiol group or selenol group by an enzyme reaction.

Examples of the substrate analog include acetylthiocholine, butylthiocholine, and a sulfur-containing lipid represented by the following structural formula.

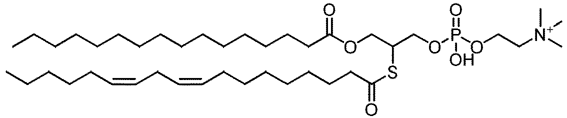

Examples of the natural substrate include S-D-lactoyl-glutathione, S-adenosylhomocysteine, and coenzyme A derivatives.

The compound represented by General Formula (I) or the salt thereof used for the detection method of the present invention is as follows.

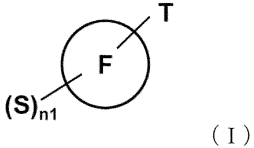

In General Formula (I), T is a reactive site that reacts with the thiol group (R-SH), the selenol group (R-SeH), or the poly sulfur atom (R-(S)n-H). Here, R is a hydrogen atom or an alkyl group. n is an integer of 1 to 5.

The reactive site is preferably selected from a nitroolefin group that may have a substituent, maleimides, an electron-deficient aryl group, and an alkyl or arylsulfonic acid group (that is, sulfonic acid substituted with an alkyl group or an aryl group, which is represented by R'-SO₃-, where R' represents an alkyl group that may have a substituent or an aryl group that may have a substituent). The electron-deficient aryl group refers to an aromatic compound substituted with one or more nitro groups, halogen groups, sulfonic acid groups, and the like. When a thiol group or the like causes an aromatic nucleophilic reaction in an electron-deficient aromatic ring, the electron-deficient aryl group can be used for detecting a target nucleophile.

When the reactive site is a nitroolefin group, reactivity with a thiol is high, which is more preferable.

S is a hydrophilic group selected from the group consisting of a sulfonic acid group, a carboxyl group, and a phosphonic acid group.

In the case of a microdevice, particularly in the case of a single-molecule measurement experimental system using a microchamber, a highly lipid-soluble device or oil is used for the construction of the chamber. Therefore, it is preferable to use a probe having high water solubility in order to prevent adsorption and leakage to the device or oil.

n1 represents an integer of 1 to 10, and is preferably 1 to 3, more preferably 1 or 2, and still more preferably 2. 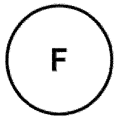 represents a fluorophore, in which molecular design is carried out so that optical properties of the entire molecule of the compound change by the reaction of the compound represented by General Formula (I) or the salt thereof with the thiol group, the selenol group, or the poly sulfur atom.

That is, an important property of the compound represented by General Formula (I) is that a reactive site of T reacts with a thiol, selenol, or a poly sulfur atom to change fluorescence characteristics, and the progress of the chemical reaction can be detected by a difference in fluorescence signal.

The fluorophore is preferably a fluorophore selected from the group consisting of a BODIPY-based fluorophore, a rhodamine-based fluorophore, a fluorescein-based fluorophore, a rhodol analog fluorophore, a cyanine-based fluorophore, a resorufin-based fluorophore, and a coumarin-based fluorophore. The fluorophore is particularly preferably a BODIPY-based fluorophore, a rhodamine-based fluorophore, a fluorescein-based fluorophore, or a rhodol analog fluorophore.

Here, either or both of S and T, or in a case where there are two or more S's and T's, some of them may be bonded to the fluorophore through a linker.

The linker is selected from the group consisting of an alkylene group (provided that one or more -CH₂- of the alkylene group may be substituted with -O-, - S-, -NH-, or -CO-), arylene (including heteroarylene), cycloalkylene, an alkoxyl group, a polyethylene glycol chain, and a group formed by arbitrarily bonding two or more groups selected from these groups. Also, in compounds of General Formulas (II), (IIa), (IIb), (IIc), and (III) or salts thereof described below, some or all of the reactive sites and the hydrophilic groups may be bonded to the fluorophore through a linker.

One preferred aspect of the compound represented by General Formula (I) or the salt thereof is a compound represented by General Formula (II) below or a salt thereof, the compound having one to five hydrophilic groups selected from the group consisting of a sulfonic acid group, a carboxyl group, and a phosphonic acid group, and having one to three reactive sites that react with the thiol group, the selenol group, or the poly sulfur atom.

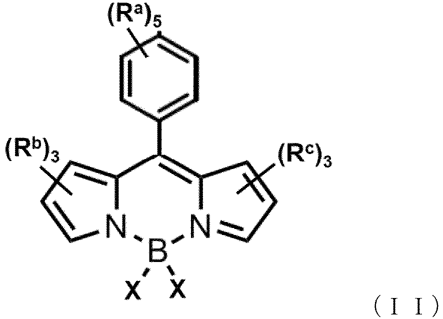

The compound represented by General Formula (II) or the salt thereof is a BODIPY-based fluorophore, and is preferable because an S/N ratio tends to increase in fluorescence increase upon the reaction with a thiol or the like, and the fluorescence intensity tends to be hardly affected by environmental factors such as pH and is also strong in light fading.

In General Formula (II), R^{a} is each independently a hydrogen atom, a monovalent substituent, the hydrophilic group, an alkyl group having the hydrophilic group and having 1 to 6 carbon atoms, or the reactive site.

R^{b} is each independently a hydrogen atom, a monovalent substituent, the hydrophilic group, an alkyl group having the hydrophilic group and having 1 to 6 carbon atoms, or the reactive site.

R^{c} is each independently a hydrogen atom, a monovalent substituent, the hydrophilic group, an alkyl group having the hydrophilic group and having 1 to 6 carbon atoms, or the reactive site.

The type of the monovalent substituent represented by R^{a}, R^{b}, and R^{c} is not particularly limited, and for example, is preferably selected from the group consisting of an alkyl group having 1 to 6 carbon atoms, an alkenyl group having 1 to 6 carbon atoms, an alkynyl group having 1 to 6 carbon atoms, an alkoxy group having 1 to 6 carbon atoms, a hydroxyl group, an alkoxycarbonyl group, a halogen atom, a carboxyl group and a derivative thereof (ester, amide, or the like), a sulfonic acid group, a phosphonic acid group, or an amino group.

These monovalent substituents may further have one or two or more arbitrary substituents. For example, one or two or more halogen atoms, hydroxyl groups, amino groups, alkoxy groups, and the like may exist in the alkyl group represented by R^{a}, R^{b}, and R^{c}, and for example, the alkyl group represented by R^{a}, R^{b}, and R^{c} may be a halogenated alkyl group, a hydroxyalkyl group, an aminoalkyl group, or the like.

X is each independently selected from a fluorine atom, an alkyl group, an alkoxy group, an aryl group, the hydrophilic group, or an alkyl group having the hydrophilic group and having 1 to 6 carbon atoms.

One preferred aspect of the compound represented by General Formula (II) or the salt thereof is a compound represented by General Formula (IIa) below or a salt thereof, the compound having one to five hydrophilic groups selected from the group consisting of a sulfonic acid group, a carboxyl group, and a phosphonic acid group, and having one to three reactive sites that react with the thiol group, the selenol group, or the poly sulfur atom.

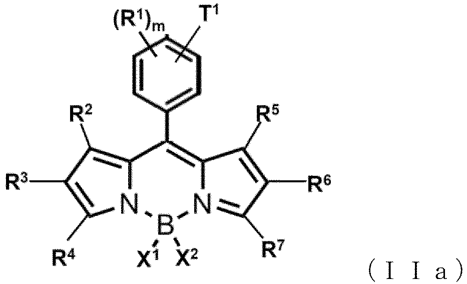

In General Formula (IIa), R¹, when present, represents the same or different monovalent substituent present on a benzene ring.

The type of the monovalent substituent represented by R¹ is not particularly limited, and for example, is preferably selected from the group consisting of an alkyl group having 1 to 6 carbon atoms, an alkenyl group having 1 to 6 carbon atoms, an alkynyl group having 1 to 6 carbon atoms, an alkoxy group having 1 to 6 carbon atoms, a hydroxyl group, an alkoxycarbonyl group, a halogen atom, a carboxyl group and a derivative thereof (ester, amide, or the like), a sulfonic acid group, a phosphonic acid group, or an amino group.

These monovalent substituents may further have one or two or more arbitrary substituents. For example, one or two or more halogen atoms, hydroxyl groups, amino groups, alkoxy groups, and the like may exist in the alkyl group represented by R¹, and for example, the alkyl group represented by R¹ may be a halogenated alkyl group, a hydroxyalkyl group, an aminoalkyl group, or the like.

In addition, the monovalent substituent may have the hydrophilic group.

m is an integer of 0 to 4, and when m is 2 or more, each R¹ may be the same or different.

In one preferred aspect of the compound represented by General Formula (IIa) or the salt thereof, m is 0 (that is, R¹ is not present).

In another preferred aspect of the compound represented by General Formula (IIa) or the salt thereof, m is 1 or 2, and R¹ is an alkoxyl group having 1 to 6 carbon atoms (preferably, methoxy group).

In General Formula (IIa), R² to R⁷ each independently represent a hydrogen atom, an alkyl group having 1 to 6 carbon atoms, the hydrophilic group, or an alkyl group having the hydrophilic group and having 1 to 6 carbon atoms.

In General Formula (IIa), T¹ is a reactive site that reacts with the thiol group, the selenol group, or the poly sulfur atom. T¹ may be bonded to two or more benzene rings.

The reactive site of T¹ is preferably a nitroolefin group or a maleimide group which may have a substituent.

In General Formula (IIa), X¹ and X² are each independently selected from a fluorine atom, an alkyl group, an alkoxy group, an aryl group, the hydrophilic group, or an alkyl group having the hydrophilic group and having 1 to 6 carbon atoms.

One preferred aspect of the compound represented by General Formula (IIa) or the salt thereof is a compound represented by General Formula (IIb) below or a salt thereof.

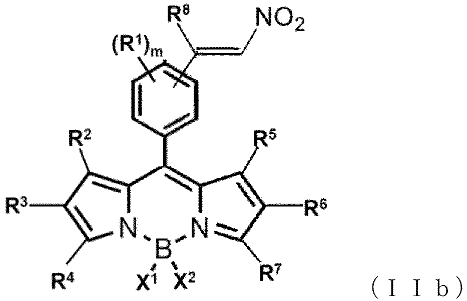

In General Formula (IIb), R¹ to R⁷, X¹, X², and m are as defined in General Formula (IIa).

R⁸ represents a hydrogen atom or a substituted or unsubstituted alkyl group having 1 to 10 carbon atoms.

In General Formula (IIb), two or more nitroolefin moieties that may have a substituent may be bonded to the benzene ring.

In General Formula (IIb), R², R⁴, R⁵, and R⁷ are each preferably a methyl group.

In General Formula (IIa), a nitroolefin moiety is preferably attached to the 2- or 4-position of the benzene ring.

In General Formula (IIb), at least one of R³ and R⁶ is a sulfonic acid group, a carboxyl group, or a phosphonic acid group.

At least one of R³ and R⁶ is preferably a carboxyl group or a phosphonic acid group, and more preferably a sulfonic acid group. The sulfonic acid group has particularly high water solubility, and when a compound having a sulfonic acid group is used, adsorption to the microdevice is not observed, and the sulfonic acid group can be suitably used for detecting enzyme activity of one molecule.

In one preferred aspect of the compound of General Formula (IIb) or the salt thereof, at least one, preferably two, of R³ and R⁶ are sulfonic acid groups.

In one preferred aspect of the compound of General Formula (IIb) or the salt thereof, at least one, preferably two, of R³ and R⁶ are carboxyl groups.

In one preferred aspect of the compound of General Formula (IIb) or the salt thereof, at least one, preferably two, of R³ and R⁶ are phosphonic acid groups.

The compound of General Formula (IIb) or the salt thereof has one to five, preferably one to three, and more preferably two hydrophilic groups selected from the group consisting of a sulfonic acid group, a carboxyl group, and a phosphonic acid group.

The compound of General Formula (IIb) or the salt thereof has one to three, preferably one or two, and more preferably one nitroolefin moiety.

One preferred aspect of the compound represented by General Formula (IIa) or the salt thereof is a compound represented by General Formula (IIc) below or a salt thereof.

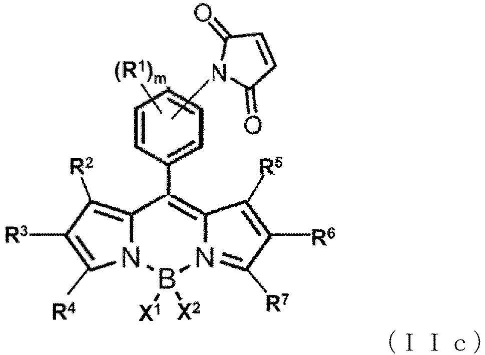

In General Formula (IIc), R¹ to R⁷, X¹, X² and m are as defined in General Formula (IIa).

In General Formula (IIc), two or more maleimide groups that may have a substituent may be bonded to the benzene ring.

In General Formula (IIc), R², R⁴, R⁵, and R⁷ are each preferably a methyl group.

In General Formula (IIc), at least one of R³ and R⁶ is a sulfonic acid group, a carboxyl group, or a phosphonic acid group.

At least one of R³ and R⁶ is preferably a carboxyl group or a phosphonic acid group, and more preferably a sulfonic acid group. The sulfonic acid group has particularly high water solubility, and when a compound having a sulfonic acid group is used, adsorption to the microdevice is not observed, and the sulfonic acid group can be suitably used for detecting enzyme activity of one molecule.

In one preferred aspect of the compound of General Formula (IIc) or the salt thereof, at least one, preferably two, of R³ and R⁶ are sulfonic acid groups.

In one preferred aspect of the compound of General Formula (IIc) or the salt thereof, at least one, preferably two, of R³ and R⁶ are carboxyl groups.

In one preferred aspect of the compound of General Formula (IIc) or the salt thereof, at least one, preferably two, of R³ and R⁶ are phosphonic acid groups.

The compound of General Formula (IIc) or the salt thereof has one to five, preferably one to three, and more preferably two hydrophilic groups selected from the group consisting of a sulfonic acid group, a carboxyl group, and a phosphonic acid group.

The compound of General Formula (IIb) or the salt thereof has one to three, preferably one or two, and more preferably one nitroolefin moiety.

One preferred aspect of the compound represented by General Formula (I) or the salt thereof is a compound represented by General Formula (III) below or a salt thereof, the compound having one to five hydrophilic groups selected from the group consisting of a sulfonic acid group, a carboxyl group, and a phosphonic acid group, and having one to three reactive sites that react with the thiol group, the selenol group, or the poly sulfur atom.

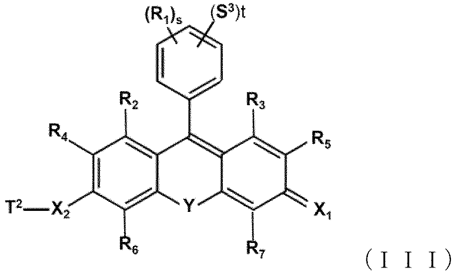

In General Formula (III), R₁, when present, represents the same or different monovalent substituent present on a benzene ring.

The type of the monovalent substituent represented by R₁ is not particularly limited, and for example, is preferably selected from the group consisting of an alkyl group having 1 to 6 carbon atoms, an alkenyl group having 1 to 6 carbon atoms, an alkynyl group having 1 to 6 carbon atoms, an alkoxy group having 1 to 6 carbon atoms, a hydroxyl group, an alkoxycarbonyl group, a halogen atom, a carboxyl group and a derivative thereof (ester, amide, or the like), a sulfonic acid group, a phosphonic acid group, or an amino group.

These monovalent substituents may further have one or two or more arbitrary substituents. For example, one or two or more halogen atoms, hydroxyl groups, amino groups, alkoxy groups, and the like may exist in the alkyl group represented by R₁, and for example, the alkyl group represented by R₁ may be a halogenated alkyl group, a hydroxyalkyl group, an aminoalkyl group, or the like.

In General Formula (III), s is an integer of 0 to 4, and when s is 2 or more, each R₁ may be the same or different.

In one preferred aspect of the compound represented by General Formula (III) or the salt thereof, m is 0 (that is, R₁ is not present).

In another preferred aspect of the compound represented by General Formula (III) or the salt thereof, m is 1 or 2, and R₁ is an alkoxyl group having 1 to 6 carbon atoms (preferably, methoxy group).

S³ is selected from a sulfonic acid group, a carboxyl group, and a phosphonic acid group.

t represents 0 to 5, preferably 1 to 4, and more preferably 1 or 2.

In General Formula (III), R² to R⁷ are each independently a hydrogen atom, an alkyl group having 1 to 6 carbon atoms, a halogen atom, the hydrophilic group, or an alkyl group having the hydrophilic group and having 1 to 6 carbon atoms.

T² is a reactive site that reacts with the thiol group, the selenol group, or the poly sulfur atom.

The reactive site of T² is preferably selected from any of the following.

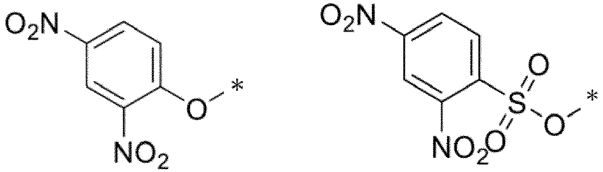

In the formula, * represents a binding site.

X¹ and X² are selected from a combination of (O,O), (O,NRs), (NR₉R₁₀,NR₈), and (NR₉R₁₀,NR₈).

Here, R₈ to R₁₀ are each independently a hydrogen atom, a substituted or unsubstituted alkyl group having 1 to 6 carbon atoms, a substituted or unsubstituted alkenyl group having 1 to 6 carbon atoms, an alkyl group having the hydrophilic group and having 1 to 6 carbon atoms, or an alkenyl group having the hydrophilic group and having 1 to 6 carbon atoms.

Y is selected from an oxygen atom, Si(Rₐ)(R_{b}), C(Rₐ)(R_{b}), P(=O)R_{c}, S(R_{d})₂, Ge(Rₐ)(R_{b}), a tellurium atom, or a bismuth atom.

Here, Rₐ and R_{b} are each independently a substituted or unsubstituted alkyl group having 1 to 6 carbon atoms or a substituted or unsubstituted aryl group.

R_{c} is a substituted or unsubstituted alkyl group having 1 to 6 carbon atoms or a substituted or unsubstituted phenyl group.

R_{d} is a substituted or unsubstituted aryl group, which is the same or different.

The alkyl group, the aryl group, and the phenyl group of Rₐ to R_{d} may be substituted with the hydrophilic group.

The compounds represented by General Formulas (I), (II), (IIa), (IIb), (IIc) and (III) (these compounds are also collectively referred to as a "compound of the present invention") can be present as acid addition salts or base addition salts. Examples of the acid addition salt include mineral acid salts such as hydrochloride, sulfate, and nitrate, and organic acid salts such as methanesulfonate, p-toluenesulfonate, oxalate, citrate, and tartrate, and examples of the base addition salt include metal salts such as sodium salt, potassium salt, calcium salt, and magnesium salt, and organic amine salts such as ammonium salt and triethylamine salt. In addition to these salts, a salt with an amino acid such as glycine may be formed. The compounds represented by General Formulas (I) and (Ia) or the salts thereof may be present as hydrates or solvates, but in the present invention, these substances can also be used.

The compound of the present invention, unless specifically stated otherwise, also includes stereoisomers thereof such as tautomers, geometric isomers (for example, E-form, Z-form, and the like), and enantiomers. That is, when one or two or more asymmetrical carbons are contained in the compound of the present invention, it can take either an (R)-form or (S)-form, each independently, in accordance with the stereochemistry of asymmetrical carbons, and sometimes exists as a stereoisomer such as an enantiomer or diastereomer of the derivative. Therefore, stereoisomers of a pure form, any mixtures of stereoisomers, racemates, and the like can be used as active ingredients of the fluorescent probe used for the detection method of the present invention, and are all encompassed within the scope of the present invention.

In the detection method of the present invention, the enzyme to be detected (target enzyme) is one or more selected from the group consisting of esterase, lipase, glyoxalase, SAH hydrolase, N-acetyltransferase, cystathionine γ-lyase, and cystathionine β-synthase, and is preferably acetylcholinesterase, phospholipase A2, or glyoxalase 2.

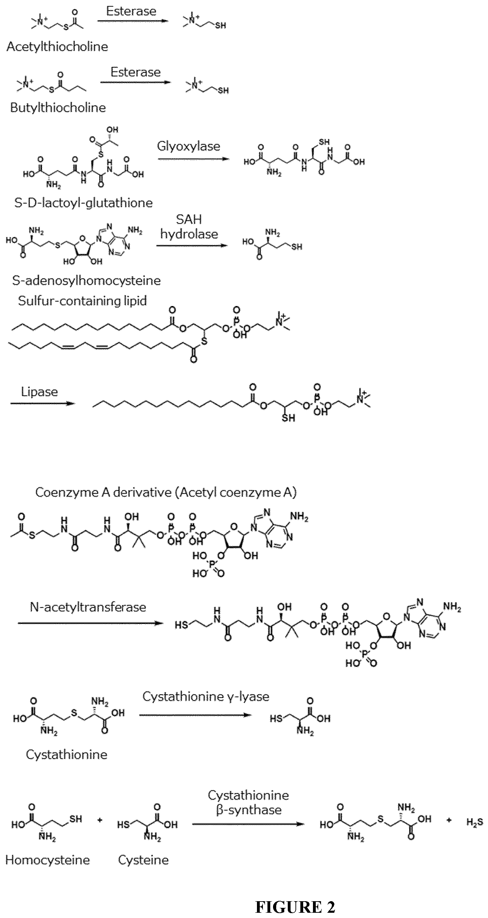

Fig. 2 illustrates a reaction formula showing how a combination of a substrate analog that can be used in the detection method of the present invention and an enzyme generates an SH group. However, the range of the detection method of the present invention is not limited to the combination of the substrate analog and the enzyme.

In the detection method of the present invention, a microdevice can be suitably used.

The microdevice includes a microchamber type device, a liposome, a droplet, and the like.

Hereinafter, a microdevice will be described using a microchamber type device as an example.

The material for the microdevice is not particularly limited, and examples thereof include glass materials, silicon, and plastics containing a dendritic polymer or copolymer. Examples of the glass materials include soda lime glass, Pyrex (registered trademark) glass, Vycor (registered trademark) glass, and quartz glass. Examples of the resin polymer include poly(vinyl chloride), poly(vinyl alcohol), poly(methyl methacrylate), poly(vinyl acetate-co-maleic anhydride), poly(dimethylsiloxane) monomethacrylate, a cyclic olefin polymer, a fluorocarbon polymer, polystyrene, polypropylene, and polyethyleneimine. Examples of the copolymer include poly(vinyl acetate-co-maleic anhydride), poly(styrene-co-maleic anhydride), poly(ethylene-coacrylic acid), or derivatives thereof.

Examples of the shape of the microdevice include a multi-well plate in which an arbitrary number of wells (for example, microwells) are arranged. The number of wells is, for example, 1 or more and 10,000,000 or less, for example, 10 or more and 500,000 or less, for example, about 100,000, or the like, per plate.

A pore diameter of the well of the microdevice may be, for example, 10 nm or more and 10 µm or less, may be, for example, 100 nm or more and 10 µm or less, and may be, for example, 1 µm or more and 10 µm or less.

A depth of the well of the microdevice may be, for example, 10 nm or more and 100 µm or less, may be, for example, 100 nm or more and 80 µm or less, and may be, for example, 200 nm or more and 70 µm or less.

When the pore diameter and depth are within the above ranges, one molecule of the target enzyme can be captured in the well, and the enzyme activity of each molecule of the enzyme in the biological sample can be detected.

The amount of the compound of the present invention contained in one well of the microdevice may be, for example, 100 nM or more and 1,000 µM or less, may be, for example, 1 µM or more and 1,000 µM or less, and may be, for example, 10 µM or more and 1,000 µM or less.

In addition, the amount of the substrate analog contained in one well of the microdevice may be, for example, 100 nM or more and 100,000 µM or less, may be, for example, 1 µM or more and 100,000 µM or less, and may be, for example, 10 µM or more and 100,000 µM or less.

As a method of using the microdevice, first, for example, a solution containing a biological sample is added to the microdevice. Sealing oil is then added dropwise to enclose the target enzyme in the biological sample in the wells of the microdevice.

In addition, the compound and the substrate analog of the present invention may be added to the solution containing a biological sample. In addition, a solution other than the enzyme may be added later.

The detection method of the present invention comprises a step of bringing the biological sample into contact with the substrate analog, and the compound of the present invention.

Examples of the biological sample include a biological sample, a biopsy sample, a body fluid sample, and an aqueous solution which are isolated from a subject. In a certain aspect, the biological sample may be a blood sample (for example, a serum sample or a plasma sample).

The contacting can be performed by mixing the biological sample with the substrate analog, and the compound of the present invention. The biological sample may be previously diluted to a predetermined magnification.

The detection method of the present invention comprises a step of observing or measuring fluorescence emitted or enhanced from a fluorescent product generated by the reaction of the compound represented by General Formula (I) or the salt thereof with the thiol group, the selenol group, or the poly sulfur atom.

When the fluorescent product emits fluorescence, the presence or absence of the fluorescence indicates the presence of the enzyme in the aqueous solution, and the intensity of the fluorescence indicates the intensity of the activity of the enzyme in the aqueous solution.

In one preferred aspect of the detection method of the present invention, contacting is performed in the presence of plasma.

The detection method of the present invention using the microdevice will be described in detail below.

### [Step 1]

First, a solution containing a biological sample is added to the microdevice including the compound of the present invention and the substrate analog. Examples of the biological sample include a biological sample, a biopsy sample, a body fluid sample, and an aqueous solution which are isolated from a subject. The biological sample may be a blood sample (for example, a serum sample or a plasma sample).

As the pH of the solution containing a biological sample, any value may be selected. For example, the pH of the solution can be set to a value close to that in the living body, and in this case, the pH may be, for example, 6.0 or more and 8.0 or less. In order to narrow down the target to a specific enzyme group, the pH of the solution can also be made acidic so as to reach an optimum pH.

A solution containing a biological sample, a solution containing the fluorescent probe of the present invention, and a solution containing a substrate analog may be separately prepared, a ratio of the biological sample, the fluorescent probe, the substrate analog may be appropriately adjusted and mixed, and the mixed solution may be added to the microdevice.

The protein concentration of the biological sample may be usually, for example, 1 pM or more and 100 pM or less, and may be, for example, 10 pM or more and 100 pM or less, as the concentration of a "target protein".

The upper limit of the total protein concentration in the sample may be, for example, about 10 µM.

As a method for measuring the protein concentration of a biological sample, for example, a method using an antibody antigen reaction (for example, ELISA method or the like) can be used as a method for measuring the concentration of a "target protein". Examples of a method for measuring the total protein concentration in a sample include colorimetric methods using a reaction between a protein and a reagent (such as a bicinchoninic acid (BCA) method, Bradford method, Lowry method, and a biuret method).

The biological sample may be diluted using various aqueous solvents or the like so as to have the above concentration. Examples of the aqueous solvent include water, physiological saline, phosphate buffered saline (PBS), tris buffered saline (TBS), and HEPES buffered saline, but are not limited thereto.

### [Step 2]

Sealing oil is then added dropwise to enclose the enzyme in the biological sample in the wells of the microdevice. The sealing oil may be any known sealing oil that is usually used for enclosing a sample in a microdevice, and examples thereof include fluorine-based oil (such as FC-40).

### [Step 3]

Next, fluorescence in the well of the microdevice is detected using a fluorescence scanner, a fluorescence microscope, or the like. The enzyme activity can be evaluated from the detected fluorescence intensity.

What enzyme is contained in the well of the microdevice in which the enzyme activity is detected can be determined, for example, by comparison with an enzyme activity pattern with a separately prepared target protein.

The detection method of the present invention can further include observing a fluorescence response using a fluorescence imaging means. As a means for observing a fluorescence response, a fluorometer having a wide measurement wavelength can be used, but the fluorescence response can also be visualized using a fluorescence imaging means capable of displaying the fluorescence response as a two-dimensional image. By using the fluorescence imaging means, the fluorescence response can be two-dimensionally visualized, so that the target enzyme can be instantly visually recognized. As a fluorescence imaging device, a device known in the art can be used. In some cases, it is also possible to detect a reaction between the sample to be measured and the fluorescent probe by a change in ultraviolet-visible absorption spectrum (for example, a change in absorbance at a specific absorption wavelength).

One aspect of the detection method of the present invention is a method for detecting an enzyme in a biological sample, the method comprising bringing two or more compounds of the present invention into contact with a plurality of enzymes.

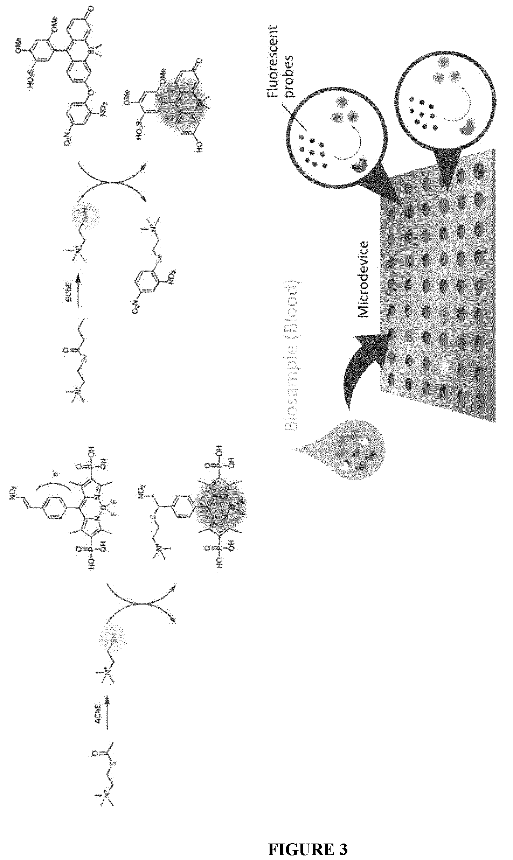

A schematic diagram of a non-limiting example of this aspect is illustrated in Fig. 3.

Fig. 3 illustrates a polychromatic assay in which AChE is detected using an acetyl thioester as a substrate analog and a compound of General Formula (IIa) as a fluorescent probe, and BChE is detected using an acetyl selenoester as a substrate analog and a compound of General Formula (III) as a fluorescent probe.

### 2. Fluorescent Probe

One embodiment of the present invention is a fluorescent probe for use in a method for detecting activity of an enzyme in a biological sample using a microdevice, the method comprising a step of bringing the biological sample into contact with a substrate analog or a natural substrate, and a compound represented by General Formula (I) below or a salt thereof,
wherein the substrate analog or the natural substrate generates a compound A having a thiol group (R-SH), a selenol group (R-SeH), or a poly sulfur atom (R-(S)n-H) (R is a hydrogen atom or an alkyl group, and n is 1 to 5) by a reaction with the enzyme,
fluorescence is observed or measured, the fluorescence being emitted or enhanced from a fluorescent product generated by the reaction of the compound represented by General Formula (I) or the salt thereof with the thiol group, the selenol group, or the poly sulfur atom, and
the substrate analog generates a compound A having a thiol group, a selenol group (R-SeH), or a poly sulfur atom (R-(S)n-H) by a reaction with the enzyme,
the fluorescent probe comprising a compound represented by General Formula (I) or a salt thereof (hereinafter, also referred to as a "fluorescent probe of the present invention").

The fluorescent probe of the present invention uses the detection method using the microdevice of the present invention, such that it is possible to detect activity of an enzyme having high substrate specificity such as acetylcholinesterase, and preferably, it is possible to detect activity of an enzyme having high substrate specificity at a single molecule level.

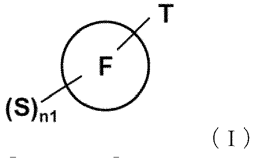

Details of the compound represented by General Formula (I) or the salt thereof (T, S, n1, and 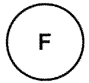 ) are as defined in detail in the detection method of the present invention.

In addition, either or both of S and T, or in a case where there are two or more S's and T's, some of them may be bonded to the fluorophore through a linker.

The linker is selected from the group consisting of an alkylene group (provided that one or more -CH₂- of the alkylene group may be substituted with -O-, - S-, -NH-, or -CO-), arylene (including heteroarylene), cycloalkylene, an alkoxyl group, a polyethylene glycol chain, and a group formed by arbitrarily bonding two or more groups selected from these groups. Also, in compounds of General Formulas (II), (IIa), (IIb), (IIc), and (III) or salts thereof described below, some or all of the reactive sites and the hydrophilic groups may be bonded to the fluorophore through a linker.

In one aspect of the fluorescent probe of the present invention, the compound represented by General Formula (I) or the salt thereof is a compound represented by General Formula (II) below or a salt thereof, the compound having one to five hydrophilic groups selected from the group consisting of a sulfonic acid group, a carboxyl group, and a phosphonic acid group, and having one to three reactive sites that react with the thiol group, the selenol group, or the poly sulfur atom.

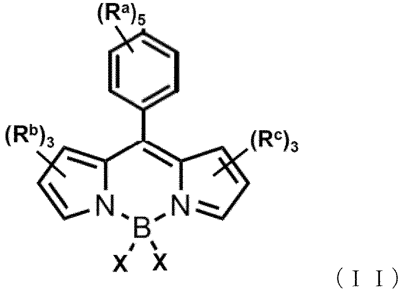

Details of the compound represented by General Formula (II) or the salt thereof (R^{a} to R^{c}, X, and the like) are as defined in detail in the detection method of the present invention.

In one aspect of the fluorescent probe of the present invention, the compound represented by General Formula (II) or the salt thereof is a compound represented by General Formula (IIa) below or a salt thereof.

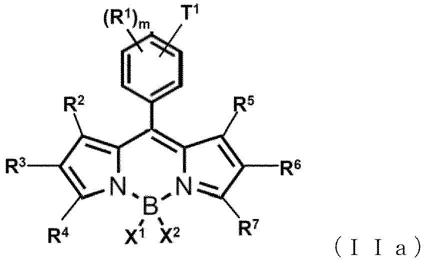

Details of the compound represented by General Formula (IIa) or the salt thereof (R¹ to R⁷, X¹, X², T¹, m, and the like) are as defined in detail in the detection method of the present invention.

In one aspect of the fluorescent probe of the present invention, the compound represented by General Formula (IIa) or the salt thereof is a compound represented by General Formula (IIb) below or a salt thereof.

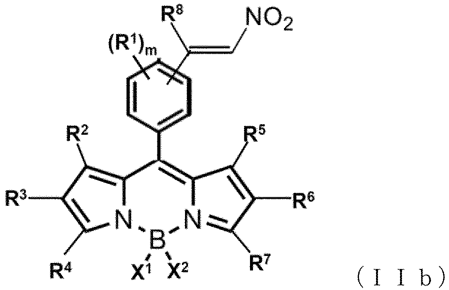

Details of the compound represented by General Formula (IIb) or a salt thereof (R¹ to R⁷, X¹, X², m, and the like) are as defined in detail in the detection method of the present invention.

In one aspect of the fluorescent probe of the present invention, the compound represented by General Formula (IIb) or the salt thereof is a compound represented by General Formula (IIc) below or a salt thereof.

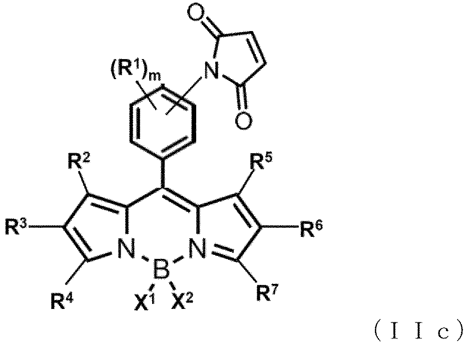

Details of the compound represented by General Formula (IIc) or a salt thereof (R¹ to R⁷, X¹, X², m, and the like) are as defined in detail in the detection method of the present invention.

In one aspect of the fluorescent probe of the present invention, the compound represented by General Formula (I) or the salt thereof is a compound represented by General Formula (III) below or a salt thereof.

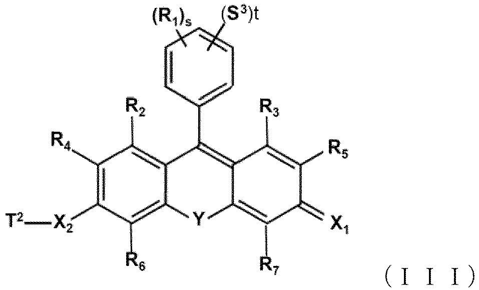

Details of the compound represented by General Formula (III) or the salt thereof (R₁ to R₇, X₁, X₂, Y, T², s, t, and the like) are as defined in detail in the detection method of the present invention.

The reactive site of T² is preferably any of the following.

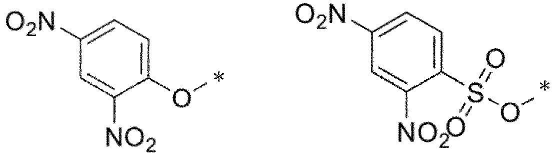

In the formula, * represents a binding site.

The substrate analog using the detection method using the fluorescent probe of the present invention preferably has a structure in which one of the oxygen atoms of the carboxylic acid ester of the substrate molecule of the enzyme to be detected is substituted with sulfur or selenium.

The substrate analog preferably has a thioester- or selenoester-like structure and generates a corresponding thiol group or selenol group by an enzyme reaction.

Examples of the substrate analog include acetylthiocholine, butylthiocholine, and a sulfur-containing lipid represented by the following structural formula.

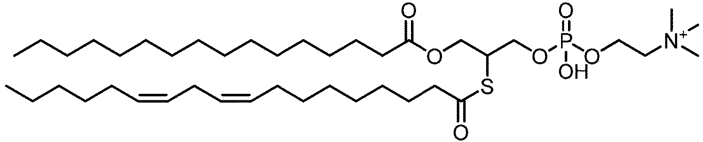

Examples of the natural substrate include S-D-lactoyl-glutathione, S-adenosylhomocysteine, and coenzyme A derivatives. Examples of the combination of the natural substrate and the enzyme using the same include natural substrate/enzyme (Japanese name): S-adenosylhomocysteine/S-adenosylhomocysteine hydrolase, S-D-lactoylglutathione/glyoxalase, coenzyme A derivative/acetyltransferase.

The fluorescent probe of the present invention can be preferably used to detect one or more enzymes selected from the group consisting of esterase, lipase, glyoxalase, SAH hydrolase, N-acetyltransferase, cystathionine γ-lyase, cystathionine β-synthase, and S-adenosylhomocysteine hydrolase, and can be more preferably used to detect acetylcholinesterase, phospholipase A2, or glyoxalase 2.

A method of using the fluorescent probe of the present invention is not particularly limited, and the fluorescent probe can be used in the same manner as a conventionally known fluorescent probe. Usually, the compound of the present invention or a salt thereof may be dissolved in an aqueous medium such as physiological saline or a buffer, a mixture of an aqueous medium and a water-miscible organic solvent such as ethanol, acetone, ethylene glycol, dimethyl sulfoxide, or dimethylformamide, or the like, and then this solution may be added to an appropriate buffer containing cells and tissues, and a fluorescence spectrum is measured. The fluorescent probe of the present invention may be used in the form of a composition in combination with an appropriate additive. For example, the fluorescent probe of the present invention can be combined with an additive such as a buffering agent, a solubilizing agent, or a pH adjusting agent.

The fluorescent probe of the present invention can be suitably used for an enzyme activity detection method using a microdevice. Therefore, by providing the fluorescent probe of the present invention and the substrate analog in a well of a microdevice, the fluorescent probe can be used for a method for detecting enzyme activity of a target hydrolase in a biological sample.

The microdevice includes a microchamber type device, a liposome, a droplet, and the like.

Details of the microdevice are as defined in detail in the detection method of the present invention.

One embodiment of the present invention is a kit for detection of an enzyme, containing a compound of General Formula (I) or a salt thereof, and a substrate analog.

In the kit, the compound of General Formula (I) or the salt thereof is usually prepared as a solution, but for example, the fluorescent probe of the present invention is provided as a composition in an appropriate form such as a mixture in a powder form, a lyophilizate, a granule, a tablet, or a liquid, and can also be dissolved in distilled water for injection or an appropriate buffer at the time of use and applied.

The kit may appropriately contain other reagents and the like as necessary. For example, additives such as a solubilizing agent, a pH adjusting agent, a buffering agent, and an isotonizing agent can be used as the additive, and the blending amount thereof can be appropriately selected by those skilled in the art.

### 3. Compounds Represented by General Formulas (IIb) and (IIc) and Salts Thereof

One embodiment of the present invention is a compound represented by General Formula (IIb) below or a salt thereof.

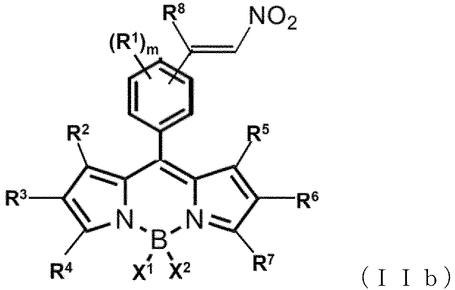

In General Formula (IIa), R¹, when present, represents the same or different monovalent substituent present on a benzene ring.

The type of the monovalent substituent represented by R¹ is not particularly limited, and for example, is preferably selected from the group consisting of an alkyl group having 1 to 6 carbon atoms, an alkenyl group having 1 to 6 carbon atoms, an alkynyl group having 1 to 6 carbon atoms, an alkoxy group having 1 to 6 carbon atoms, a hydroxyl group, an alkoxycarbonyl group, a halogen atom, a carboxyl group and a derivative thereof (ester, amide, or the like), a sulfonic acid group, a phosphonic acid group, or an amino group.

These monovalent substituents may further have one or two or more arbitrary substituents. For example, one or two or more halogen atoms, hydroxyl groups, amino groups, alkoxy groups, and the like may exist in the alkyl group represented by R¹, and for example, the alkyl group represented by R¹ may be a halogenated alkyl group, a hydroxyalkyl group, an aminoalkyl group, or the like.

In addition, the monovalent substituent may have the hydrophilic group.

m is an integer of 0 to 4, and when m is 2 or more, each R¹ may be the same or different.

In one preferred aspect of the compound represented by General Formula (IIb) or the salt thereof, m is 0 (that is, R¹ is not present).

In another preferred aspect of the compound represented by General Formula (IIb) or the salt thereof, m is 1 or 2, and R¹ is an alkoxyl group having 1 to 6 carbon atoms (preferably, methoxy group).

In General Formula (IIb), R⁸ represents a hydrogen atom or a substituted or unsubstituted alkyl group having 1 to 10 carbon atoms.

In General Formula (IIb), R² to R⁷ are each independently a hydrogen atom, an alkyl group having 1 to 6 carbon atoms, the hydrophilic group, or an alkyl group having the hydrophilic group and having 1 to 6 carbon atoms.

In General Formula (IIb), R², R⁴, R⁵, and R⁷ are each preferably a methyl group.

In General Formula (IIb), two or more nitroolefin moieties that may have a substituent may be bonded to the benzene ring.

In General Formula (IIa), a nitroolefin moiety is preferably attached to the 2- or 4-position of the benzene ring.

In General Formula (IIb), at least one of R³ and R⁶ is a sulfonic acid group, a carboxyl group, or a phosphonic acid group.

At least one of R³ and R⁶ is preferably a carboxyl group or a phosphonic acid group, and more preferably a sulfonic acid group. The sulfonic acid group has particularly high water solubility, and when a compound having a sulfonic acid group is used, adsorption to the microdevice is not observed, and the sulfonic acid group can be suitably used for detecting enzyme activity of one molecule.

In one preferred aspect of the compound of General Formula (IIb) or the salt thereof, at least one, preferably two, of R³ and R⁶ are sulfonic acid groups.

In one preferred aspect of the compound of General Formula (IIb) or the salt thereof, at least one, preferably two, of R³ and R⁶ are carboxyl groups.

In one preferred aspect of the compound of General Formula (IIb) or the salt thereof, at least one, preferably two, of R³ and R⁶ are phosphonic acid groups.

The compound of General Formula (IIb) or the salt thereof has one to five, preferably one to three, and more preferably two hydrophilic groups selected from the group consisting of a sulfonic acid group, a carboxyl group, and a phosphonic acid group.

The compound of General Formula (IIb) or the salt thereof has one to three, preferably one or two, and more preferably one nitroolefin moiety.

One embodiment of the present invention is a compound represented by General Formula (IIc) below or a salt thereof.

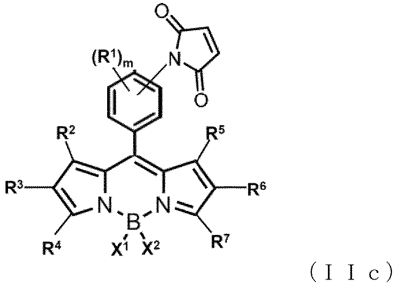

In General Formula (IIc), R¹ to R⁷, X¹, X² and m are as defined in General Formulas (IIa) and (IIb).

In General Formula (IIc), two or more maleimide groups that may have a substituent may be bonded to the benzene ring.

In General Formula (IIc), R², R⁴, R⁵, and R⁷ are each preferably a methyl group.

In General Formula (IIc), at least one of R³ and R⁶ is a sulfonic acid group, a carboxyl group, or a phosphonic acid group.

At least one of R³ and R⁶ is preferably a carboxyl group or a phosphonic acid group, and more preferably a sulfonic acid group. The sulfonic acid group has particularly high water solubility, and when a compound having a sulfonic acid group is used, adsorption to the microdevice is not observed, and the sulfonic acid group can be suitably used for detecting enzyme activity of one molecule.

In one preferred aspect of the compound of General Formula (IIc) or the salt thereof, at least one, preferably two, of R³ and R⁶ are sulfonic acid groups.

In one preferred aspect of the compound of General Formula (IIc) or the salt thereof, at least one, preferably two, of R³ and R⁶ are carboxyl groups.

In one preferred aspect of the compound of General Formula (IIc) or the salt thereof, R³

The compounds represented by General Formulas (IIb) and (IIc) can be present as acid addition salts or base addition salts. Examples of the acid addition salt include mineral acid salts such as hydrochloride, sulfate, and nitrate, and organic acid salts such as methanesulfonate, p-toluenesulfonate, oxalate, citrate, and tartrate, and examples of the base addition salt include metal salts such as sodium salt, potassium salt, calcium salt, and magnesium salt, and organic amine salts such as ammonium salt and triethylamine salt. In addition to these salts, a salt with an amino acid such as glycine may be formed. The compounds represented by General Formulas (I) and (Ia) or the salts thereof may be present as hydrates or solvates, but in the present invention, these substances can also be used.

The compounds represented by General Formulas (IIb) and (IIc), unless specifically stated otherwise, also include their stereoisomers such as tautomers, geometric isomers (for example, E-form, Z-form, and the like), and enantiomers. That is, when one or two or more asymmetrical carbons are contained in the compound of the present invention, it can take either an (R)-form or (S)-form, each independently, in accordance with the stereochemistry of asymmetrical carbons, and sometimes exists as a stereoisomer such as an enantiomer or diastereomer of the derivative. Therefore, stereoisomers of a pure form, any mixtures of stereoisomers, racemates, and the like can be used as active ingredients of the fluorescent probe used for the detection method of the present invention, and are all encompassed within the scope of the present invention.

Non-limiting examples of the compounds of General Formulas (IIb) and (IIc) or the salts thereof are shown below.

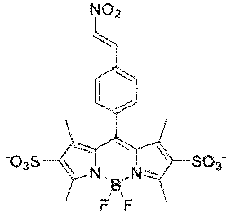

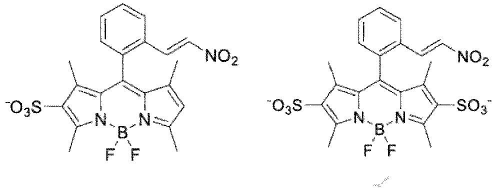

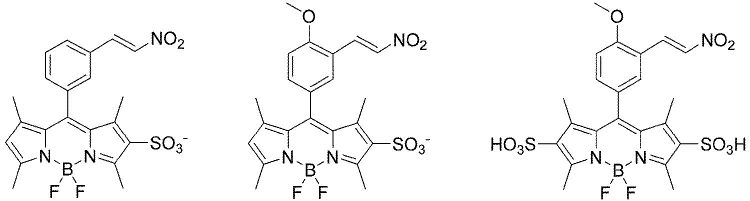

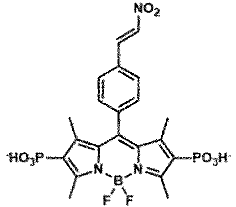

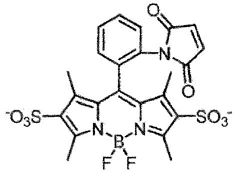

The following compounds included in the compound represented by General Formula (III) are also within the scope of the present invention.

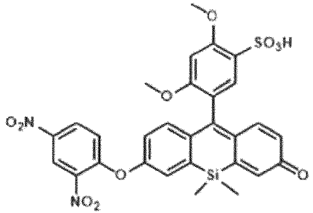

The method for producing the compounds represented by General Formulas (IIb), (IIc), and (III) or the salts thereof is not particularly limited, and a synthesis method for representative compounds among the compounds included above are specifically shown in Examples of the present specification. Those skilled in the art can produce the compounds represented by General Formulas (IIa), (IIc), and (III) by suitably changing or modifying the starting raw materials, reaction reagents, reaction conditions, and the like as necessary while referring to Examples and the following schemes of the present specification.

### EXAMPLES

Hereinafter, the present invention will be described with reference to Examples; however, the present invention is not limited thereto.

### 1. Materials and Methods

### (1) Materials

As reagents and solvents, special grade products supplied from Tokyo Chemical Industry Co., Ltd., Wako Pure Chemical Industries, Ltd., Sigma-Aldrich, Dojindo Laboratories Co., Ltd., KANTO CHEMICAL CO., INC., and WATANABE CHEMICAL INDUSTRIES, LTD. were used without purification. Enzymes were purchased from Sigma-Aldrich and used without purification.

### (2) Measurement Device

NMR spectra were measured using JEOL JNM-LA400 device at 400 MHz for ¹HNMR and 100 MHz for ¹³CNMR.

Mass spectra (MS) were measured with JEOL JMS-T100 LC AccuToF (ESI).

Preparative HPLC was performed with Inertsil ODS-3 (10.0 x 250 mm) column (GL Sciences Inc.) using an HPLC system consisting of a pump (PU-2080, JASCO Corporation) and a detector (MD-2015 or FP-2025, JASCO Corporation). An eluent A (H₂O containing 0.1% TFA), an eluent B (80% acetonitrile containing 0.1% TFA and 20% H₂O), an eluent C (H₂O containing 100 mM triethylammonium acetate), and an eluent D (80% acetonitrile containing 100 mM triethylammonium acetate and 20% H₂O) were used for HPLC purification.

LC-MS analysis was performed with Waters Acquity UPLC (class H)/QDa quadrupole MS analyzer or Acquity UPLC (class H)/Xevo TQD quadrupole MS/MS analyzer equipped with Acquity UPLC BEH C18 column (Waters Corporation). An eluent A (H₂O containing 0.1% formic acid), an eluent B (80% acetonitrile containing 0.1% formic acid and 20% H₂O), an eluent C (H₂O containing 10 mM ammonium formate), and an eluent D (80% acetonitrile containing 10 mM ammonium formate and 20% H₂O) were used.

Column chromatography using silica gel was performed with an HPLC system (Yamazen Smart Flash EPCLC AI-5805 (Tokyo, Japan)). Reverse-phase MPLC purification was performed with Isolera One (Biotage) equipped with 30 g of SNAP Ultra C (Biotage).

### (3) UV-Vis Absorption and Fluorescence Spectroscopy

Ultraviolet-visible spectra were obtained with UV-1800 of Shimadzu Corporation. Studies were performed by fluorescence spectroscopy using Hitachi F7000. A slit width was 5 nm for both excitation and emission. A photomultiplier tube voltage was 400 V

### (4) Digital Enzyme Measurement with Microdevice

An enzyme stock solution was diluted with an assay buffer containing a fluorescent probe. Next, 40 µL of a reaction mixture was added to the microdevice (Simoa disk; Quanterx Corporation). Next, 80 µL of FC-70 (Sigma-Aldrich) was introduced into the microdevice to wash off the excess reaction mixture and form W/O droplets. A fluorescence signal of the catalytically produced fluorescent dye was measured under a fluorescence microscope, and the enzyme activity in the chamber was measured.

### (5) Fluorescence Imaging of Microdevice

Fluorescence images were acquired using a 20x dry objective (Plan Apo 20×), an sCMOS camera (ORCA-FusionC14440, Hamamatsu Photonics K.K.), a white LED lighting unit (X-Cite Xylis, Opto Science, Inc.), and a fluorescence microscope (Ti2, Nikon Corporation) equipped with a motorized stage. In the analysis, measurement was performed using a solution containing sSiR (10 µM) as an internal standard and the focus was adjusted using fluorescence. Images were acquired in tile scan mode at full focus. FITC (mirror = 510 nm, Ex. = 460 to 500 nm, Em. = 510 to 560 nm) was used as an excitation filter, and mCherry (mirror = 600 nm, Ex. = 550 to 590 nm, Em. = 608 to 683 nm) was used as an emission filter.

### (6) Image Processing

Images were processed using GA3 module of NIS Elements software (Nikon Corporation). First, all fluorescence images were background corrected using rolling ball correction (3 µm). Next, ROI was selected by bright spot detection using mCherry filter (diameter = 3 µm, contrast = 500), and irregular fluorescence spots from fluorescent debris or bubbles were omitted by expanding the ROI and removing overlapping ROIs. The fluorescence signal was acquired as the average of the signals from the center of each ROI. Data were processed using Excel or Kaleidagraph software to create a histogram and a scatter diagram.

### (7) Enzymatic Assay

An enzymatic assay was performed in phosphate buffered saline (pH 7.4). Half area 384 well plates (Corning 3677) were used for the assay (reaction quantity of 20 µL). Fluorescence was detected with a plate reader with appropriate filter settings, EnVision 2103 Multilabel Reader (PerkinElmer, Inc.).

### (8) Plasma Samples and Code of Ethics

Plasma samples were collected as part of Health Science Basic Research Promotion Project funded by Health and Labour Sciences Research Grant from National Cancer Center (Tokyo, Japan), National Hospital Organization Osaka National Hospital (Osaka, Japan), Osaka Medical and Pharmaceutical University (Takatsuki, Japan), National Institute of Biomedical Innovation, and Ministry of Health, Labour and Welfare, and P-CREATE 36-39 project of Japan Agency for Medical Research and Development (AMED) at Kagoshima Prefectural Total Health Center. Ethical approval for this study was obtained from Central Ethics Committee of Nippon Medical School (M-2021-002) and Ethics Committee of Nippon Medical School (A-2020-032 and A-2020-044).

### 1. Synthesis and Characteristic Evaluation of Compounds

### [Synthesis Example 1]

### Synthesis of Compound 1 (2-sulfo-nitroolefin BODIPY)

A compound 1 was synthesized according to Scheme 1 below.

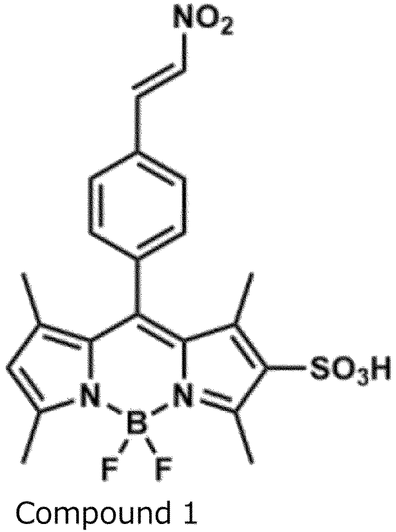

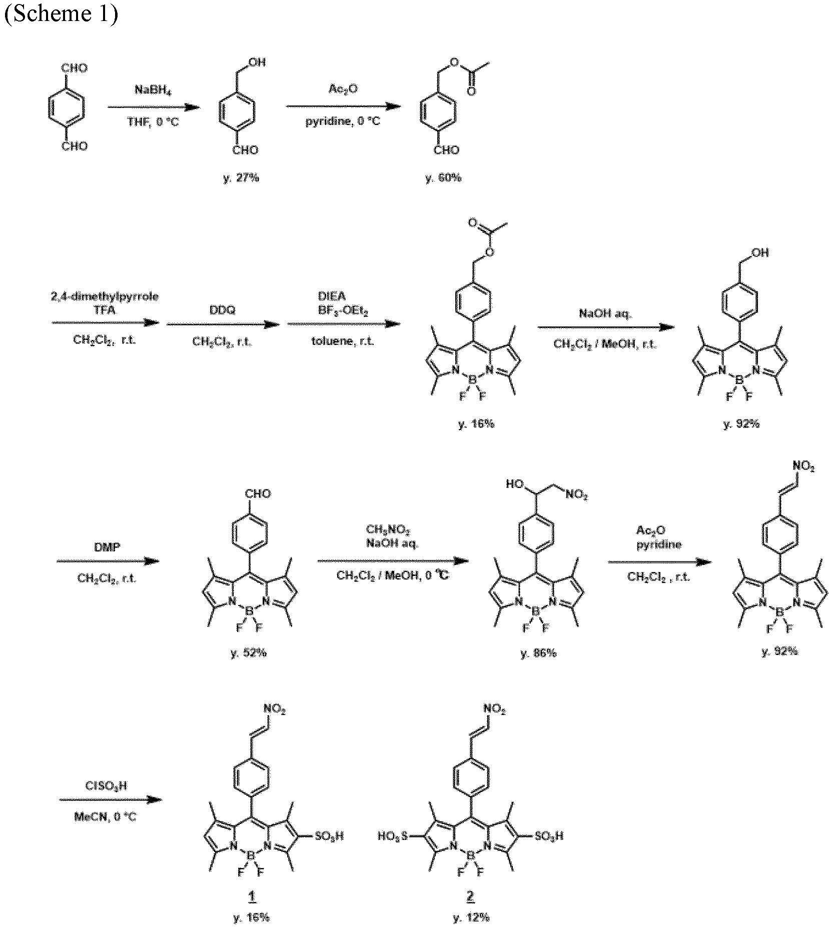

The synthesis up to nitroolefin BODIPY was based on the literature (In Situ Evaluation of Kinetic Resolution Catalysts for Nitroaldol by Rationally Designed Fluorescence Probe (J. Org. Chem. 2011, 76, 10, 3616-3625 Publication Date: March 3, 2011).

An MeCN solution (3 mL) of the nitroolefin BODIPY (7 mg, 0.018 mmol) was cooled to 0°C and chlorosulfuric acid (3.13 mg, 0.027 mmol) was added. The mixture was stirred at 0°C for 10 minutes. Subsequently, the reaction was quenched with H₂O containing 2 M triethylammonium acetate. The reaction mixture was purified by HPLC (eluent A: 0.1% TFA-containing H₂O, eluent B: 0.1% TFA-containing 80% acetonitrile and 20% H₂O; gradient: A/B = 95/5 to 0/100, 15 minutes) to obtain a compound 1 (red solid, 1.4 mg, yield of 17%).

¹H-NMR (400 MHz, DMSO-d6):δ 8.33 (d,J=13.7Hz,1H), 8.23 (d,J=13.7Hz,1H), 8.06 (d, J=8.0Hz,2H), 7.52 (d,J=8.0Hz,2H), 6.21(s, 1H), 2.65(s, 3H), 2.46 (s, 3H),1.57(s, 3H),1.32(d,3H); HRMS (ESI-) :Calcd. for [M-H]-, 474.11065 ; Found, 474.10862 (-2.04 mDa)

The HPLC chromatogram after purification was as follows (eluent A: 0.1% TFA-containing H₂O, eluent B: 0.1% TFA-containing 80% acetonitrile and 20% H₂O; gradient: A/B = 80/20 to 20/80, 60 minutes; flow rate: 1.0 mL/min; detection: absorbance at 490 nm).

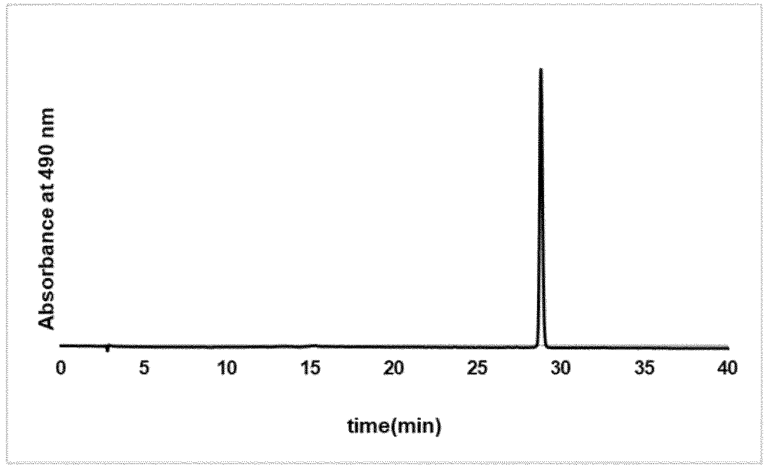
Absorbance at 490 nm
0 5 10 15 20 25 30 35 40
time(min)

### [Synthesis Example 2]

### Synthesis of Compound 2 (2,6-sulfo-nitroolefin BODIPY)

A compound 2 was synthesized according to Scheme 1.

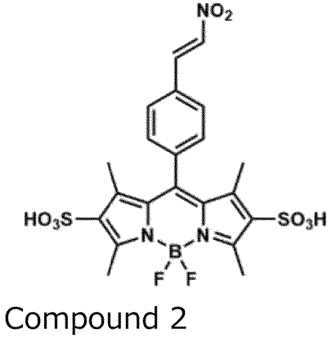

An MeCN (3 mL) solution of 8-(p-nitroolefin)-BODIPY (10.7 mg, 0.027 mmol) was cooled to 0°C, and then chlorosulfuric acid (11.04 mg, 6.3 µL, 0.094 mmol) was added. The mixture was stirred at 0°C for 10 minutes. Subsequently, the mixture was quenched with H₂O containing 2 M triethylammonium acetate. The reaction mixture was purified by HPLC (eluent A: 0.1% TFA-containing H₂O, eluent B: 0.1% TFA-containing 80% acetonitrile and 20% H₂O; gradient: A/B = 95/5 to 0/100, 15 minutes) to obtain a compound 2 (red solid, 1.8 mg, yield of 12%).

¹H-NMR (400MHz,DMSO-d6):δ 8.34 (d,J=13.7Hz,1H),8.23 (d,J=13.7Hz,1H),8.06 (d, J=7.8Hz,2H),7.52(d,J=8.2Hz,2H),2.65(s,6H),1.53 (s,6H); HRMS (ESI-) :Calcd. for [M-H]-, 554.06747; Found, 554.06589 (-1.58 mDa)

The HPLC chromatogram after purification (eluent: A/B = 95/5 to 5/95 in 4 minutes) is shown. Absorbance at 500 nm was detected. An eluent A (H₂O containing 100 mM triethylammonium acetate) and an eluent B (80% acetonitrile containing 100 mM triethylammonium acetate and 20% H₂O) were used.

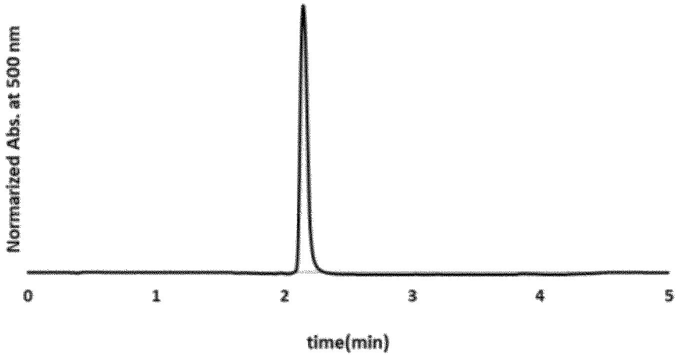
Normarized Abs. at 500 nm
0 1 2 3 4 5
time(min)

### [Synthesis Example 3]

### Synthesis of Compound 4 (2-CO₂H Nitroolefin BODIPY)

A compound 4 was synthesized according to Scheme 2 below.

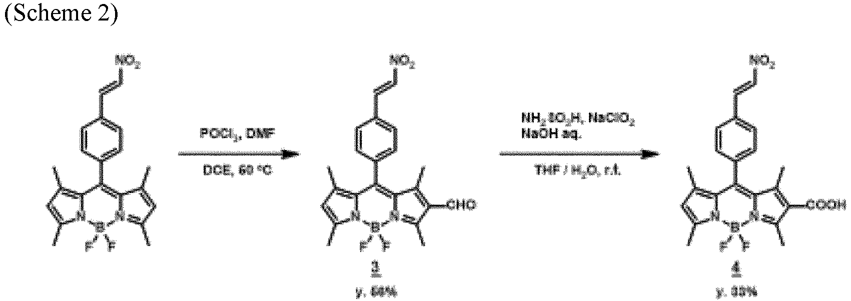

### (1) Synthesis of Compound 3 (2-Formylnitroolefin BODIPY)

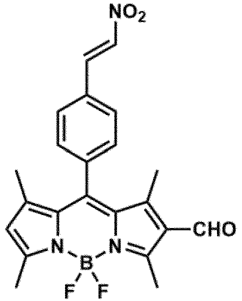

Phosphorus oxychloride (2 mL) was added dropwise to dimethylformamide (2 mL) in an ice batch under an Ar atmosphere for 5 minutes. The solution was allowed to warm to room temperature and stirred for 30 minutes. 8-(p-Nitroolefin)-BODIPY (20 mg, 0.050 mmol) was added to dichloroethane (10 mL), the temperature was increased to 50°C, and the reaction mixture was stirred for 30 minutes. Thereafter, the reaction mixture was cooled to room temperature and quenched with a saturated aqueous NaHCO₃ solution. Next, an aqueous layer was extracted with CH₂Cl₂. The combined organic extracts were washed with brine, dried with anhydrous Na₂SO₄, and filtered, and the solvent was removed under reduced pressure. The obtained residue was purified by MPLC (silica gel, 80/20 to 0/100 hexane/AcOEt) to obtain a compound 3 (red solid, 12 mg, yield of 56%).

¹H-NMR (400 MHz,CDCl3) :δ10.02 (s,1H), 8.08 (d, J=13.7 Hz,1H), 7.75 (d, J=7.8Hz, 2H), 7.69 (d, J=13.7Hz, 1H), 7.44 (d, J=8.2Hz, 2H), 6.19 (s, 1H), 2.83 (s, 3H), 2.63 (s, 3H), 1.68 (s, 3H), 1.45 (s, 3H); HRMS (ESI-) :Calcd. for [M-H]-, 422.14875; Found, 422.15008 (1.33mDa)

### (2) Synthesis of Compound 4 (2-CO₂H Nitroolefin BODIPY)

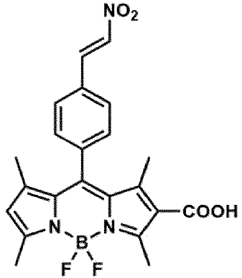

To a mixture of THF (6 mL) and water (2 mL), the compound 3 (14 mg, 0.033 mmol) was added, and then, NaClO₂ (6 mg, 0.066 mmol) and NH₂SO₃H (29 mg, 0.3 mmol) were added. The reaction mixture was stirred for 30 minutes, diluted with AcOEt, and then washed with a saturated aqueous Na₂S₂O₃ solution. An organic layer was washed with brine, dried with anhydrous Na₂SO₄, and filtered, and the solvent was removed under reduced pressure. The obtained residue was purified by MPLC (silica gel, 80/20 to 0/100 hexane/AcOEt) to obtain a compound 4 (pink-red solid, 5 mg, yield of 34%).

¹H-NMR (400MHz, DMSO-d6) :δ 8.33 (d, J=13.7Hz, 1H), 8.21 (d, J=13.7Hz, 1H), 8.04 (d, J=8.2Hz, 2H), 7.53 (s, 2H), 6.35 (s, 1H), 2.64 (s, 3H), 2.48 (s, 3H), 1.57 (s, 3H), 1.34 (s, 3H); HRMS (ESI-) :Calcd. for [M-H]-, 438.14367; Found, 438.14181 (-1.86 mDa)

### [Synthesis Example 4]

### Synthesis of Compound 9 (2,6-diCO₂H Nitroolefin BODIPY)

A compound 9 was synthesized according to Scheme 3 below.

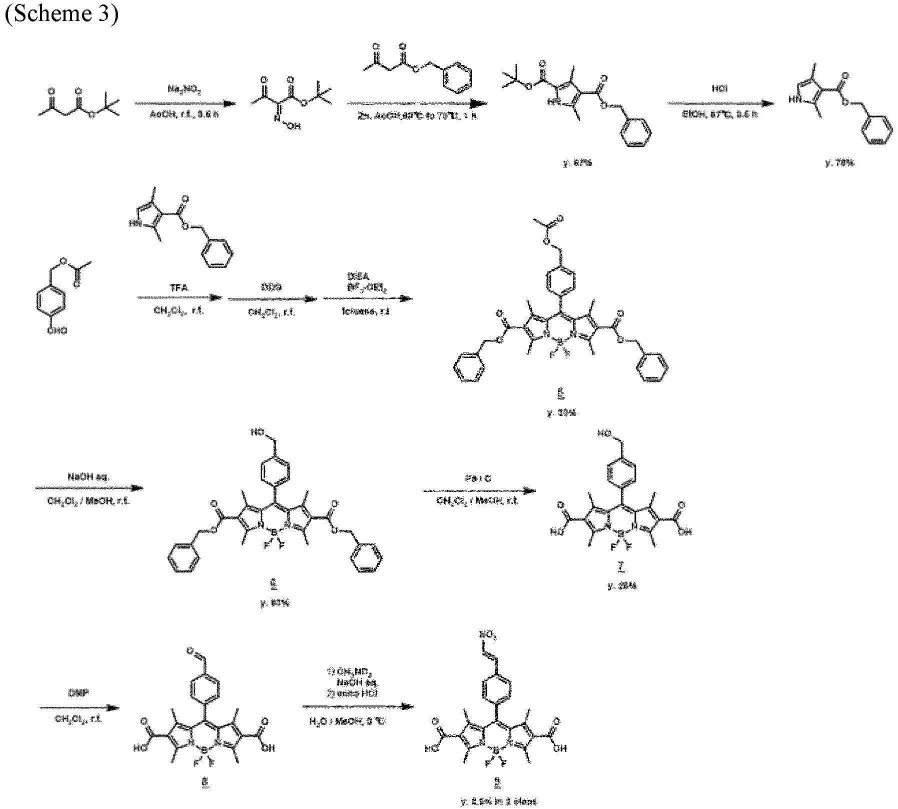

### (1) Synthesis of Compound 5 (2,6-diCO2Bzl Acetoxymethyl BODIPY)

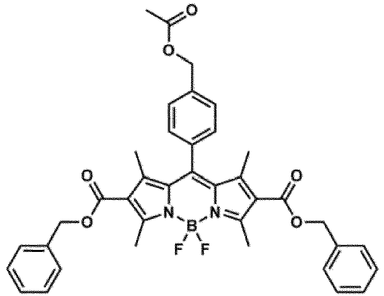

2,4-Dimethyl-1H-pyrrole-3-carboxylic acid ethyl ester (250 mg, 1.09 mmol) and methyl (4-formylphenyl)acetate (96 mg, 0.54 mmol) were dissolved in CH₂Cl₂ (100 mL). Trifluoroacetic acid (0.1 mL) was added under an Ar atmosphere, and the mixture was stirred at room temperature for 12 hours. 2,3-Dichloro-5,6-dicyano-p-benzoquinone (DDQ; 122 mg, 0.54 mmol) was added, the mixture was stirred for 30 minutes, and then, the reactant was washed three times with brine, dried with Na₂SO₄, filtered, and evaporated. The remaining red solid was purified with a filtration column (alumina). The obtained crude dipyrromethene was mixed with toluene and N,N-diisopropyl-N-ethylamine (DIEA; 2 mL). BF₃-OEt₂ (1.5 mL) was added dropwise under an Ar atmosphere, and the mixture was stirred at room temperature for 1 hour. Next, AcOEt was added, and the mixture was washed with brine, dried with Na₂SO₄, filtered, and evaporated. The obtained residue was purified by MPLC (silica gel, 80/20 to 0/100 hexane/AcOEt) to obtain a compound 5 (orange solid, 120 mg, yield of 33%).

¹H-NMR (400MHz, CDCl3) :δ7.51-7.24 (m, 14H), 5.26 (s, 4H), 5.21 (s, 2H), 2.82 (s, 6H), 2.17 (s, 3H), 1.64 (s, 6H); ¹³C-NMR (101MHz, CDCl3) :δ170.9, 164.1, 160.0, 147.9, 145.5, 138.0, 135.9, 134.1, 131.5, 129.0, 128.7, 128.4, 128.0, 122.3, 66.3, 65.5, 21.1, 15.3, 14.0; HRMS (ESI+) :Calcd. for [M+H]+, 665.26345 ; Found, 665.26588 (2.43 mDa)

### (2) Synthesis of Compound 6 (2,6-diCO₂Bzl Hydroxymethyl BODIPY)

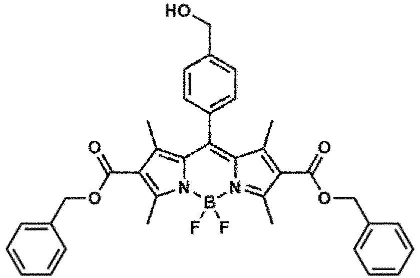

The compound 5 (200 mg, 0.298 mmol) was dissolved in CH₂Cl₂/methanol at 2:1 (20 mL). An aqueous 2N NaOH solution (2 mL) was added, and the solution was stirred at room temperature for 3 hours. The reaction mixture was diluted with CH₂Cl₂, washed with an aqueous 2N HCl solution and brine, dried with anhydrous Na2SO4, and then filtered, and the solvent was removed under reduced pressure. The obtained residue was purified by MPLC (silica gel, 80/20 to 0/100 hexane/AcOEt) to obtain compound 6 (orange solid, 170 mg, yield of 93%).

¹H-NMR (400MHz, CDCl3) :δ7.54-7.24 (m, 14H), 5.31 (s, 1H), 5.27 (s, 4H), 4.84 (s, 2H), 2.83 (s, 6H), 1.65 (s, 6H); ¹³C-NMR (101MHz, CDCl3) :δ164.1, 159.9, 148.0, 145.9, 142.8, 135.9, 133.4, 131.6, 128.7, 128.4, 127.9, 127.8, 122.2, 66.3, 64.7, 15.3, 14.1; HRMS (ESI+) :Calcd. for [M+H]+, 623.25288; Found, 623.25423 (1.35 mDa)

### (3) Synthesis of Compound 7 (2,6-diCO₂H Hydroxymethyl BODIPY)

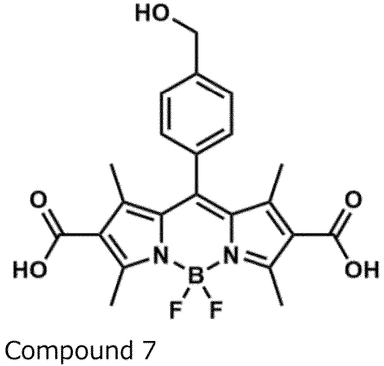

The compound 6 (40 mg, 0.64 mmol) was dissolved in CH₂Cl₂/methanol at 2:1 (20 mL). After a small amount of 10%Pd-C was added, the mixture was stirred under a H₂ atmosphere for 3 hours. When TLC monitoring (silica; CH₂Cl₂-0.1% (v/v) AcOH) showed complete product formation, Pd-C was removed by filtration, washed with brine, dried with anhydrous Na₂SO₄, and then filtered, and the solvent was removed under reduced pressure. The obtained residue was purified by HPLC (eluent A: H₂O containing 100 mM triethylammonium acetate, eluent B: 80% acetonitrile containing 100 mM triethylammonium acetate and 20% H₂O; gradient: A/B = 95/5 to 0/100, 15 minutes). The obtained solution was desalted, evaporated under reduced pressure, and lyophilized to obtain a compound 7 (red solid, 8 mg, yield of 28%).

¹H-NMR (400MHz, DMSO-d6):δ7.53 (d, J=8.2Hz, 2H), 7.36 (d, J=8.2Hz, 2H), 5.42 (s, 1H), 4.63 (s, 2H), 2.70 (s, 6H), 1.59 (s, 6H); HRMS (ESI-) :Calcd. for [M-H]-, 441.14333; Found, 441.14091 (-2.42 mDa)

### (4) Synthesis of Compound 8 (2,6-diCO₂H Formyl BODIPY)

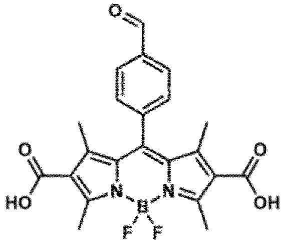

Dess-Martin periodinane (6.1 mg, 0.027 mmol) was added to an MeCN (2 mL) solution of the compound 7 (8 mg, 0.018 mmol) containing 10% DMSO. The mixture was stirred at room temperature for 3 hours and evaporated. A crude purified compound 8 was used in the next step without further purification.

### (5) Synthesis of Compound 9 (2,6-diCO₂H Nitroolefin BODIPY)

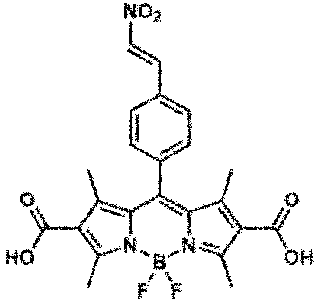

A mixture of nitromethane (10 µL, 0.2 mmol) and the compound 8 in methanol (0.5 mL) was stirred at 0°C. An aqueous 2N NaOH solution (1 mL) was added for 30 minutes. Stirring was continued at 0°C for a further 30 minutes. The mixture was diluted with water (3 mL) and poured into crushed ice containing 3 mL of concentrated HCl. After stirring for 30 minutes, the reactant was evaporated. The remaining liquid was purified by HPLC (eluent A: H₂O containing 100 mM triethylammonium acetate, eluent B: 80% acetonitrile containing 100 mM triethylammonium acetate and 20% H₂O; gradient: A/B = 95/5 to 0/100, 15 minutes). The obtained solution was desalted, evaporated under reduced pressure, and lyophilized to obtain a compound 9 (red solid, 3.4 mg, 2-step yield of 3.3%).

¹H-NMR (400 MHz, MeOD):δ8.15 (d, J=13.7Hz, 1H), 8.04(d, J = 13.7 Hz, 1H), 7.93 (d, J=7.8Hz, 2H), 7.50 (d, J=7.8Hz, 2H), 2.75 (s, 6H), 1.68 (s, 6H); HRMS (ESI-) :Calcd. for [M-H]-, 482.13350; Found, 482.13229 (-1.21 mDa)

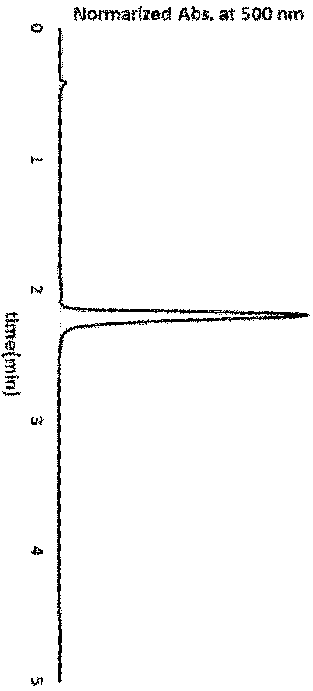

The HPLC chromatogram after purification (eluent: A/B = 95/5 to 5/95 in 4 minutes) is shown. Absorbance at 500 nm was detected. An eluent A (H₂O containing 0.01 M ammonium formate) and an eluent B (80% acetonitrile containing 0.01 M ammonium formate and 20% H₂O) were used.

### [Reference Synthesis Example 1]

### Synthesis of Compound 13 (2,6-diPO₃H₂-BODIPY)

A compound 13 was synthesized according to Scheme 4 below.

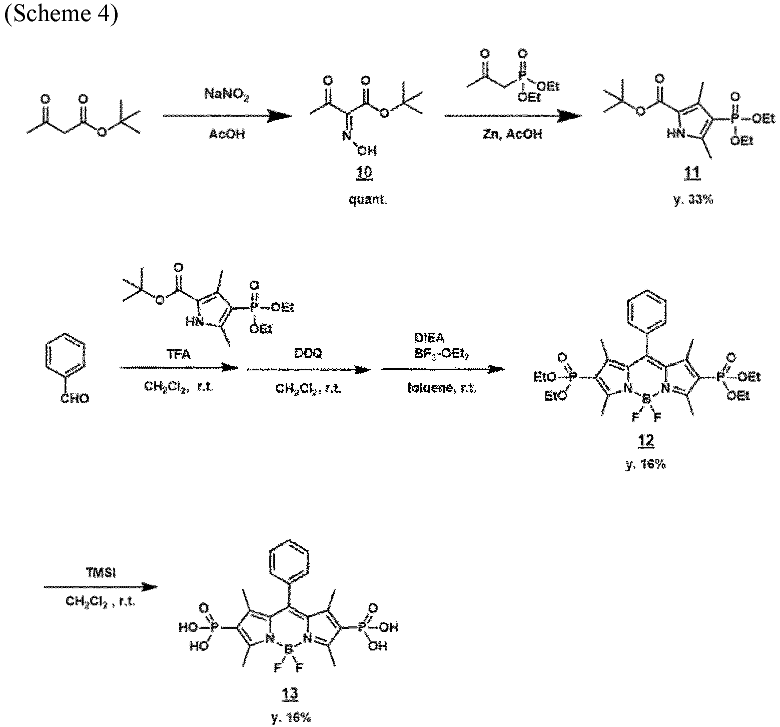

### (1) Synthesis of Compound 10 (tert-Butyl 2-hydroxymino-3-oxobutyrate)

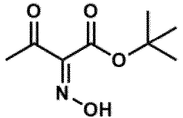

tert-Butyl-3-oxobutanoate (8 mL, 48 mmol) was dissolved in acetic acid (20 mL) in a round-bottom flask and cooled to about 10°C in an ice bath. Sodium nitrite (3.6 g, 52 mmol) was added for 30 minutes while maintaining the temperature at 15°C or lower. The cold water bath was removed, and the mixture was stirred at room temperature for 3.5 hours. The reaction mixture was diluted with CH₂Cl₂, washed with a saturated aqueous NaHCO₃ solution and brine, dried with anhydrous Na₂SO₄, and then filtered, and the solvent was removed under reduced pressure. In the next step, the crude purified compound 10 was used without further purification.

### (2) Synthesis of Compound 11

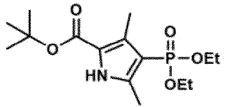

A part of zinc dust (1.3 g, 20 mmol) was added to a stirred mixture of diethyl(2-oxopropyl)phosphonate (3.5 mL, 20 mmol). The mixture was heated to 60°C. At this temperature, the crude purified compound 10 (3.6 g, 20 mmol) in acetic acid (3 mL) slowly precipitated. Thereafter, the temperature was increased to 75°C and maintained for 1 hour. The reaction mixture was diluted with CH₂Cl₂, washed with brine, dried with anhydrous Na₂SO₄, and then filtered, and the solvent was removed under reduced pressure. The obtained residue was purified by HPLC (eluent A: 0.1% TFA-containing H₂O, eluent B: 0.1% TFA-containing 80% acetonitrile and 20% H₂O; gradient: A/B = 95/5 to 0/100, 15 minutes) to obtain a compound 11 (light brown oil, 458 mg, yield of 7%).

¹H-NMR (400MHz, CDCl3) :δ9.01 (s, 1H), 4.12-4.01 (m, 4H), 2.49 (s, 2H), 2.41 (s, 2H), 1.57 (s, 9H), 1.33-1.30 (m, 6H); ¹³C-NMR (101MHz, CDCl3) ; δ160.9, 140.5, 131.3, 120.6, 107.5, 81.4, 61.7, 28.5, 16.3, 13.5, 11.8; HRMS (ESI-) :Calcd. for [M-H]-, 330.14703; Found, 330.14519 (-1.85 mDa)

### (3) Synthesis of Compound 12 (2,6-diPO₃Et₂-BODIPY)

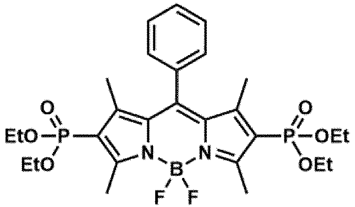

The compound 11 (214 mg, 0.64 mmol) and benzaldehyde (35 µL, 0.32 mmol) were dissolved in CH₂Cl₂ (100 mL). Trifluoroacetic acid (0.1 mL) was added under an Ar atmosphere, and the mixture was stirred at room temperature for 12 hours. DDQ (72.6 mg, 0.32 mmol) was added, the mixture was stirred for 30 minutes, and the reactant was washed three times with brine, dried with Na₂SO₄, filtered, and evaporated. The remaining red solid was purified with a filtration column (alumina). The obtained crude dipyrromethene was dissolved in toluene and DIEA (2 mL). BF₃-OEt₂ (1.5 mL) was added dropwise under an Ar atmosphere, and the mixture was stirred at room temperature for 1 hour. Next, AcOEt was added, and the mixture was washed with brine, dried with Na₂SO₄, filtered, and evaporated. The crude product was purified by MPLC (silica gel, 80/20 to 0/100 hexane/AcOEt) to obtain a compound 12 (orange solid, 32 mg, yield of 16%).

¹H-NMR (400MHz, CDCl3) :δ7.54 (m, 3H), 7.26 (m, 2H), 4.07 (m, 8H), 2.81 (s, 6H), 1.63 (s, 6H), 1.30 (m, 12H); ¹³C-NMR (101 MHz, CDCl3):δ161.2, 161.0, 151.3, 151.2, 145.4, 134.2, 132.4, 132.2, 129.9, 129.8, 127.5, 118.9, 116.9, 61.8, 61.7, 16.4, 14.7, 13.9; HRMS (ESI+) :Calcd. for [M+H]+, 597.22662; Found, 597.22734

### (0.72 mDa)

### (4) Synthesis of Compound 13 (2,6-diPO₃H₂-BODIPY)

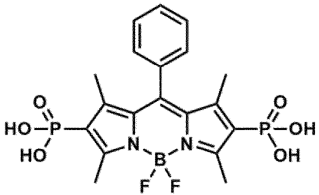

The compound 12 (32 mg, 0.05 mmol) was dissolved in CH₂Cl₂ (2 mL). Trimethylsilyl iodide (66 µL, 0.45 mmol) was added under an Ar atmosphere, and the mixture was stirred at room temperature for 15 minutes. An aqueous Na₂S₂O₃ solution was added. After stirring for 30 minutes, the reactant was evaporated. The remaining liquid was purified by HPLC (eluent A: H₂O containing 100 mM triethylammonium acetate, eluent B: 80% acetonitrile containing 100 mM triethylammonium acetate and 20% H₂O; gradient: A/B = 95/5 to 0/100, 15 minutes). The obtained solution was desalted, evaporated under reduced pressure, and lyophilized to obtain a compound 13 (orange solid, 4 mg, yield of 16%).

¹H-NMR (400MHz, DMSO-d6):δ7.51- 7.22 (m, 4H), 4.62 (s, 2H),, 2.43 (s, 6H), 1.48 (s, 6H)

### [Synthesis Example 5]

### Synthesis of Compound 17 (2,6-diPO₃H₂ Nitroolefin BODIPY)

A compound 17 was synthesized according to Scheme 4 below.

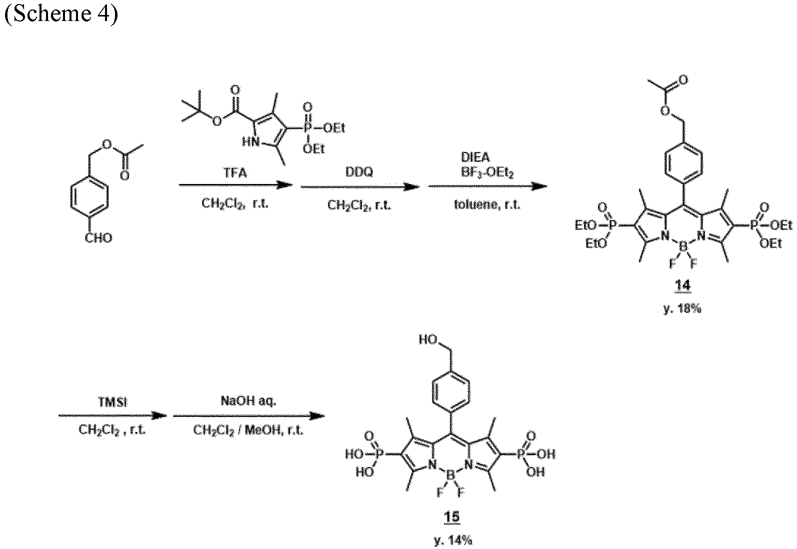

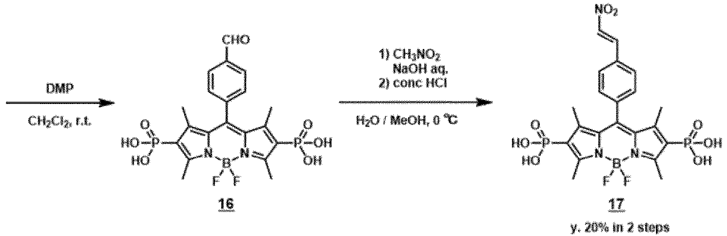

### (1) Synthesis of Compound 14 (2,6-diPO₃Et₂ Acetoxymethyl BODIPY)

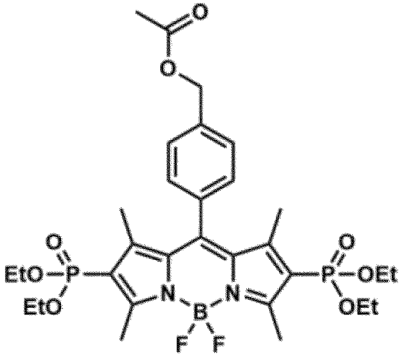

The compound 11 (379 mg, 1.15 mmol) and methyl (4-formylphenyl)acetate (101.5 mg, 0.57 mmol) were dissolved in CH₂Cl₂ (200 mL). Trifluoroacetic acid (0.1 mL) was added under an Ar atmosphere, and the mixture was stirred at room temperature for 12 hours. DDQ (129.4 mg, 0.57 mmol) was added, the mixture was stirred for 30 minutes, and the reactant was washed three times with brine, dried with Na₂SO₄, filtered, and evaporated. The remaining red solid was purified with a filtration column (alumina). The obtained crude dipyrromethene was dissolved in toluene and DIEA (2 mL). BF₃-OEt₂ (1.5 mL) was added dropwise under an Ar atmosphere, and the mixture was stirred at room temperature for 1 hour. Next, AcOEt was added, and the mixture was washed with brine, dried with Na₂SO₄, filtered, and evaporated. The crude product was purified by MPLC (silica gel, 80/20 to 0/100 hexane/AcOEt) to obtain a compound 14 (orange solid, 158 mg, yield of 18%).

¹H-NMR (400MHz, CDCl3) :δ7.53 (d, J=8.2Hz, 2H), 7.28 (d, J=8.2Hz, 2H), 5.20 (s, 2H), 4.16-3.98 (m, 8H), 2.82 (s, 6H), 2.16 (s, 3H), 1.63 (s, 6H), 1.32-1.28(m, 12H); ¹³C-NMR(101MHz, CDCl3) :δ161.2, 161.0, 151.3, 151.2, 145.5, 143.4, 133.0, 132.5, 132.3, 127.9, 127.6, 118.7, 116.7, 64.3, 61.9, 61.8, 29.8, 16.4, 16.3, 14.7, 14.0; HRMS (ESI+) :Calcd. for [M+H]+, 669.24775; Found, 669.24394 (-3.80 mDa)

### (2) Synthesis of Compound 15 (2,6-diPO₃H₂ Hydroxymethyl BODIPY)

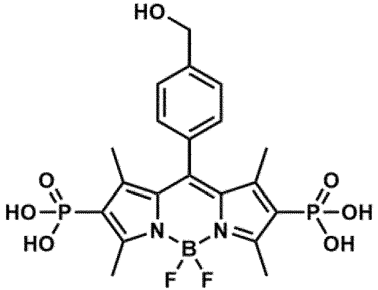

The compound 14 (50 mg, 0.074 mmol) was dissolved in CH₂Cl₂ (2 mL). Trimethylsilyl iodide (97 µL, 0.66 mmol) was added under an Ar atmosphere, and the mixture was stirred at room temperature for 15 minutes. Thereafter, the mixture was quenched with a saturated aqueous Na₂S₂O₃ solution. Next, 2N NaOHaq. (1 mL) was added. After stirring for 30 minutes, the reactant was evaporated. The remaining liquid was purified by HPLC (eluent A: H₂O containing 100 mM triethylammonium acetate, eluent B: 80% acetonitrile containing 100 mM triethylammonium acetate and 20% H₂O; gradient: A/B = 95/5 to 0/100, 15 minutes). The obtained solution was desalted, evaporated under reduced pressure, and lyophilized to obtain a compound 15 (orange solid, 5 mg, yield of 14%).

¹H-NMR (400MHz, DMSO-d6):δ7.51 (d, J = 8.2 Hz, 2H), 7.22 (m, 2H), 4.62 (s, 2H), 2.43 (s, 6H), 1.48 (s, 6H); HRMS (ESI-) :Calcd. for [M-H]-, 513.09634; Found, 513.09675 (0.41 mDa)

### (3) Synthesis of Compound 16 (2,6-diPO₃H₂ Formyl BODIPY)

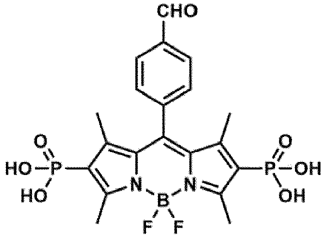

Dess-Martin periodinane (6.2 mg, 0.015 mmol) was added to a solution of the compound 15 (5 mg, 0.01 mmol) in MeCN (2 mL) containing 10% DMSO. The mixture was stirred at room temperature for 3 hours and evaporated to obtain a crude purified compound 16, and the crude purified compound 16 was used in the next step without further purification.

### (4) Synthesis of Compound 17 (2,6-diPO₃H₂ Nitroolefin BODIPY)

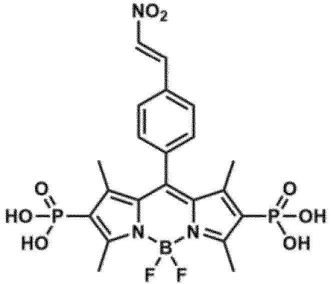

A mixture of nitromethane (1 µL, 0.02 mmol) and the compound 16 was stirred in methanol (0.5 mL) at 0°C. An aqueous 2N NaOH solution (1 mL) was added for 30 minutes. Stirring was continued at 0°C for a further 30 minutes. The mixture was diluted with water (3 mL) and poured into crushed ice containing 3 mL of concentrated HCl. After stirring for 30 minutes, the reactant was evaporated. The remaining liquid was purified by HPLC (eluent A: H₂O containing 100 mM triethylammonium acetate, eluent B: 80% acetonitrile containing 100 mM triethylammonium acetate and 20% H₂O; gradient: A/B = 95/5 to 0/100, 15 minutes). The obtained solution was desalted, evaporated under reduced pressure, and lyophilized to obtain a compound 17 (orange solid, 1.2 mg, 2-step yield of 20%).

¹H-NMR (400MHz, METHANOL-d4):δ8.16 (d, J=13.7Hz, 1H), 8.04 (d, J=13.7Hz, 1H), 7.93 (d, J=8.2Hz, 2H), 7.49 (d, J=7.8Hz, 2H), 2.72 (s, 6H), 1.67 (s, 6H); HRMS (ESI-) :Calcd. for [M-H]-, 554.08650; Found, 554.08845 (1.95 mDa)

### [Example 1]

### (1) Optical Properties of Compounds 1 and 2

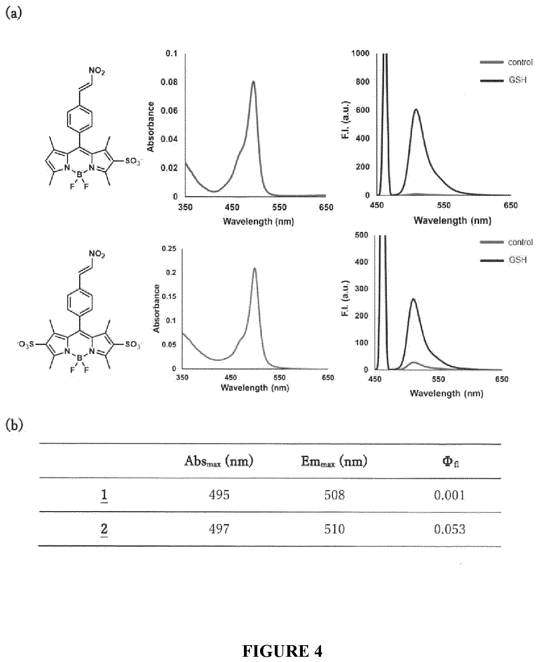

The optical properties of the compound 1 obtained in Synthesis Example 1 and the compound 2 obtained in Synthesis Example 2 were evaluated. The results are illustrated in Fig. 4.

Fig. 4(a) illustrates absorption spectra and emission spectra of the compounds 1 and 2, respectively. These spectra were measured at pH 7.4 in a 0.1 M sodium phosphate buffer containing 0.1% DMSO as a co-solvent. Glutathione (GSH; 100 µM) was added as a model thiol. The fluorescence spectra were measured under excitation at 460 nm.

Fig. 4(b) illustrates absorbance, maximum emission wavelength, and quantum yield of the compounds 1 and 2. All data were measured in a 0.1 M sodium phosphate buffer at pH 7.4.

### (2) Microdevice Compatibility of Compounds 1 and 2

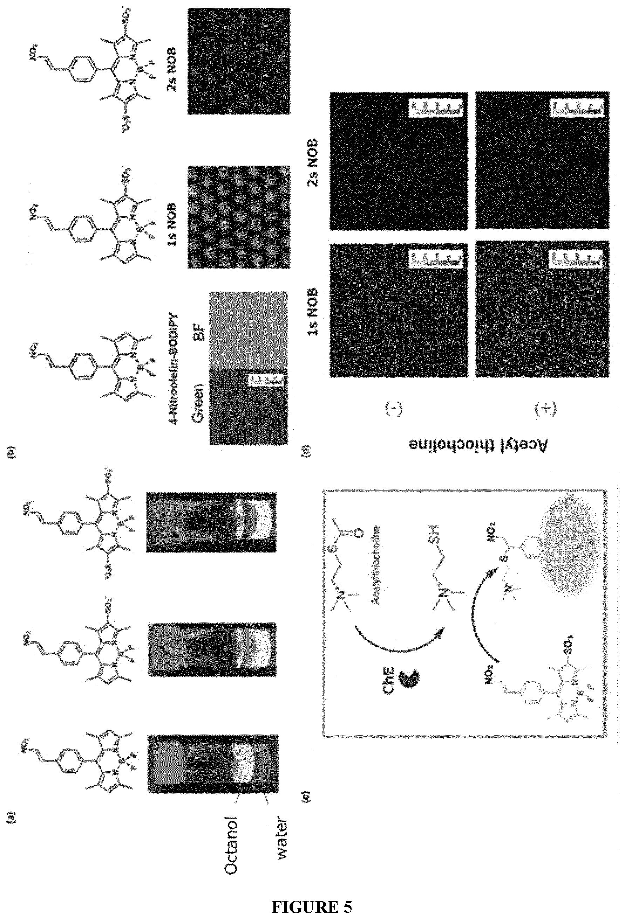

Fig. 5 illustrates the results of examination of the microdevice compatibility of the compounds 1 and 2.

Fig. 5(a) illustrates a photograph of a state in which each of three kinds of probes containing 2- and 6-position unsubstituted BODIPY and GSH are supplemented with octanol and water. Fig. 5(b) illustrates a fluorescence image of a microdevice equipped with a probe and reacted with a thiol.

Fig. 5(c) illustrates a schematic diagram of a coupled assay of an acetylthiocholine system for detection of cholinesterase (ChE) activity.

Fig. 5(d) illustrates fluorescence images of a microdevice containing human plasma that was loaded with a fluorescent probe (30 µM) in HEPES-buffer (10 mM, pH 7.4, containing 0.1% CHAPS) and incubated at 37°C for 1.5 hours. The upper diagram illustrates the results of a plasma sample containing no acetylthiocholine iodide (1,000-fold dilution), and the lower diagram illustrates the results of a plasma sample containing acetylthiocholine iodide (1 mM) (1,000-fold dilution).

It was confirmed that the 2- and 6-position unsubstituted BODIPY had high lipid solubility and was dissolved in oil, such that a fluorescence signal was not obtained, and on the other hand, leakage of both the monosulfonic acid form and the disulfonic acid form enclosed in the well in the microdevice to the oil was suppressed. On the other hand, in the case the monosulfonic acid form, a fluorescence image of the reaction vessel was observed in a donut shape (Fig. 4(b)). This is considered to be a result of adsorption of the probe to the inner wall of the reaction vessel formed of CYTOP due to insufficient water solubility of the monosulfonic acid form. Such adsorption was not observed in the disulfonic acid form.

### [Example 2]

### (1) Optical Properties of Compound 4 and Compound 9

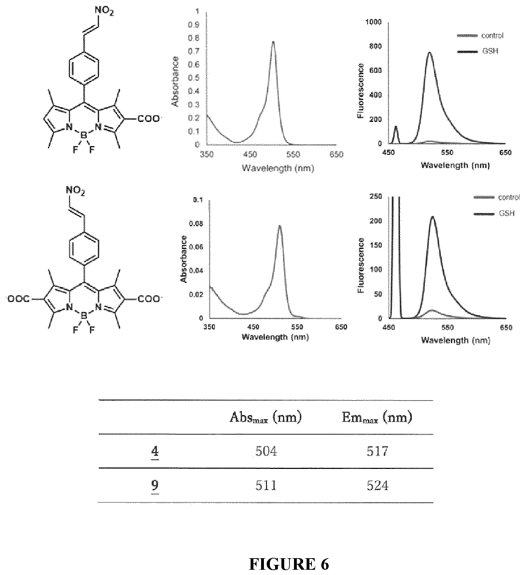

The optical properties of the compound 4 obtained in Synthesis Example 3 and the compound 9 obtained in Synthesis Example 4 were evaluated. The results are illustrated in Fig. 6.

Fig. 6 illustrates absorption spectra and emission spectra of the compounds 4 and 9, respectively. These spectra were measured at pH 7.4 in a 0.1 M sodium phosphate buffer containing 0.1% DMSO as a co-solvent. GSH (100 µM) was added as a model thiol. The fluorescence spectra were measured under excitation at 460 nm. In addition, the maximum wavelengths of absorption and emission of each probe are shown.

### (2) Microdevice Compatibility of Compounds 4 and 9

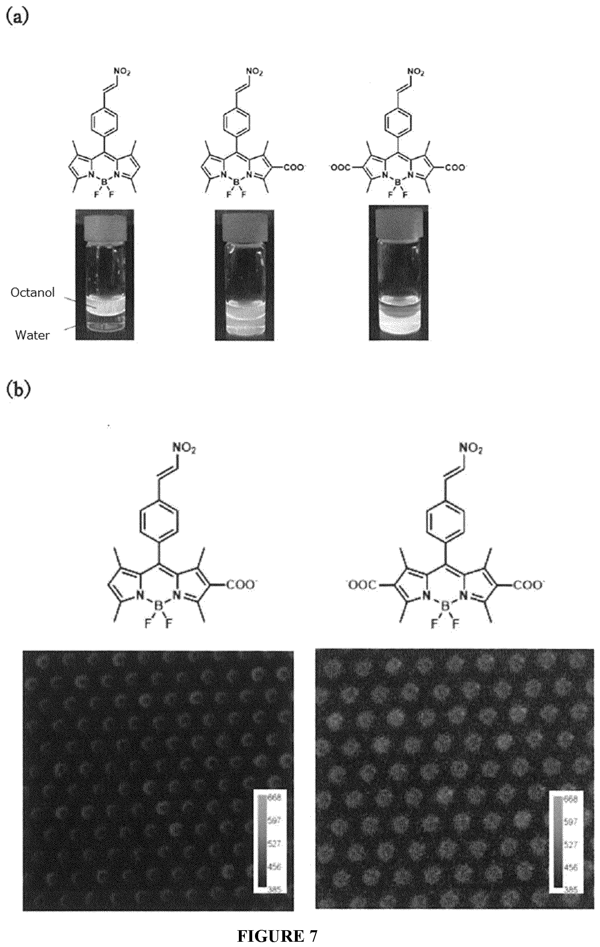

Fig. 7 illustrates the results of examination of the microdevice compatibility of the compounds 4 and 9.

Fig. 7(a) illustrates a photograph (water-octanol distribution experiment) of a state in which each probe and GSH are supplemented with octanol and water. Fig. 7(b) illustrates a fluorescence image of a microdevice equipped with a probe and reacted with a thiol.

For the compounds 4 and 9, the S/N was improved as compared with Example 1, but the adsorption to the well was the same result as in Example 1. The monocarboxylic acid form was particularly highly lipid-soluble, and in the water-octanol distribution experiment, it was observed that a large amount of the monocarboxylic acid form was distributed to octanol (lipid-soluble solvent) side, and even in the dicarboxylic acid form, distribution to octanol was partially observed.

### [Example 3]

### (1) Optical Properties of Compound 17

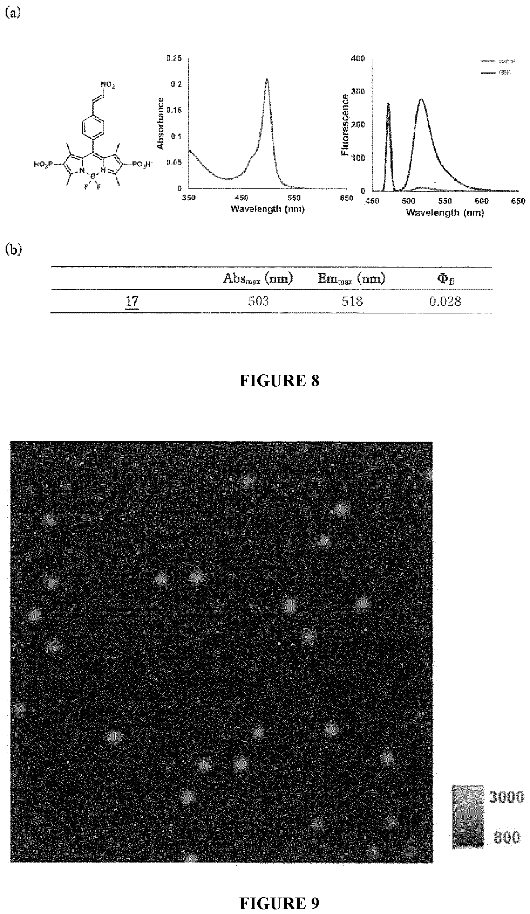

Fig. 8(a) illustrates absorption spectra and emission spectra of the compound 17, respectively. These spectra were measured at pH 7.4 in a 0.1 M sodium phosphate buffer containing 0.1% DMSO as a co-solvent. GSH (100 µM) was added as a model thiol. The fluorescence spectra were measured under excitation at 470 nm.

Fig. 8(b) illustrates absorbance, maximum emission wavelength, and quantum yield of the probe. All data were measured in a 0.1 M sodium phosphate buffer at pH 7.4.

The S/N in the buffer at pH 7.4 was significantly higher than that of the disulfonic acid form (compound 2). This is considered to be because, in BODIPY in an equilibrium of PO₃H⁻ and PO₃²⁻ (the abundance ratio is estimated to be approximately 1:3 at pH 7.4), the electron withdrawing property is lower than that of sulfonic acid, and thus, d-PeT sufficiently occurs in a state before reaction with a thiol.

### (2) Microdevice Compatibility of Compound 17

Fig. 8 illustrates the results of examination of the microdevice compatibility of the compound 17.

Fig. 9 illustrates a fluorescence image of the microdevice in which human plasma (3,000-fold dilution) was mixed with the fluorescent probe (compound 17; 30 µM) and placed in HEPES buffer (10 mM, pH 7.4, containing 0.1% CHAPS) with or without acetylthiocholine iodide (1 mM). The image was acquired after incubation at 25°C for 30 minutes.

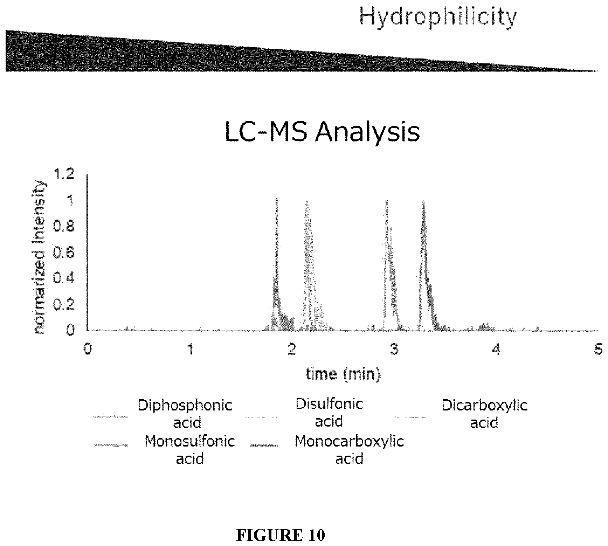

Due to the high water solubility, no adsorption to the microdevice was observed, and it was found to be available for detection of one molecule of enzyme activity. When the comparison of the water solubilities of the probes developed in Examples 1 to 3 was evaluated by the difference in the elution time of LC-MS (it is known that the water solubility can be estimated by the ease of retention on the C18 carrier), it was suggested again that the water solubility was greatly improved in the diphosphonic acid form (Fig. 10).

Fig. 10 illustrates the analysis of a mixture of five compounds by LC-MS and the extracted ion chromatogram of each compound shown with the display color. The results of the diphosphonic acid form, the dicarboxylic acid form, the disulfonic acid form, the monosulfonic acid form, and the monocarboxylic acid form are shown from the left.

The compound was eluted with a linear gradient of A/B = 100/0 at 0.5 minutes and 100/0 to 0/100 at 3 minutes detected at 500 nm (eluent A: H₂O containing 10 mM ammonium formate; eluent B: acetonitrile 80%, H₂O 20% containing 10 mM ammonium formate).

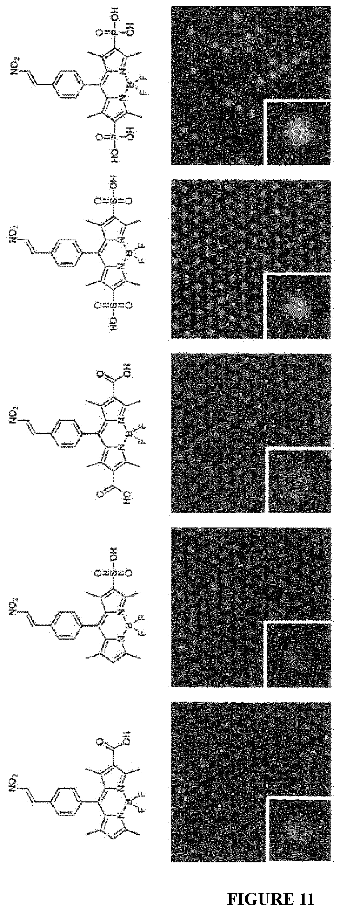

Fig. 11 illustrates the results of the microdevice compatibility of the compounds 1, 2, 4, 9, and 17 performed in Examples 1 to 3.

### [Example 4]

Next, the compound 17 (2,6-diPO₃H₂ nitroolefin BODIPY) examined in Example 3 was used to verify whether or not the enzyme activity in human blood was detected using the microdevice.

First, various parameters were changed, and the following conditions were set as optimal conditions.

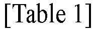

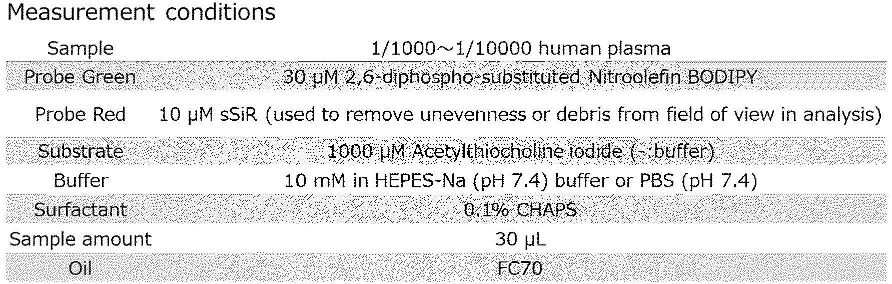

### (1) Detection of Enzyme in Blood

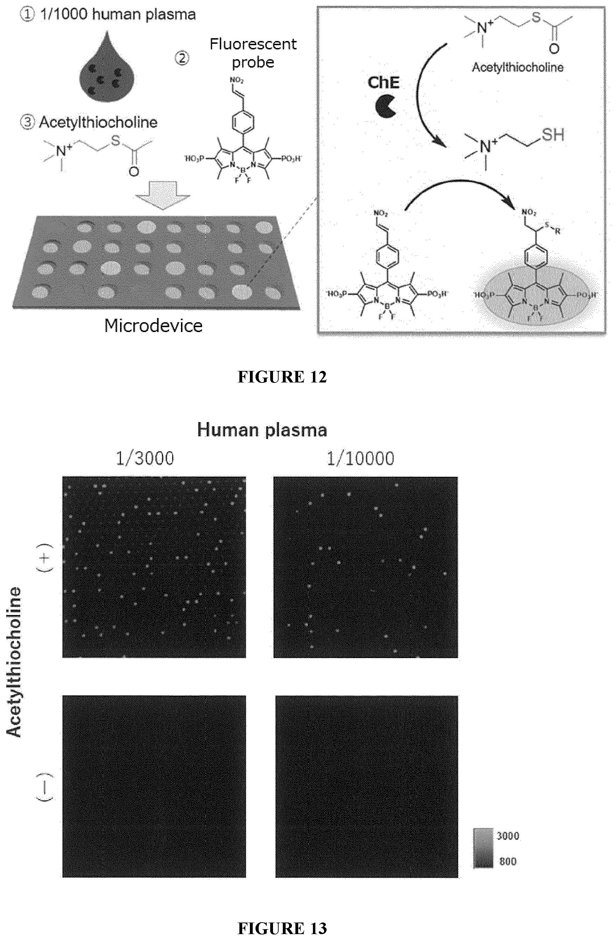

Fig. 12 illustrates a schematic diagram of detection of an enzyme in blood using a microdevice.

The measured results are illustrated in Fig. 13. Fig. 13 is a fluorescence image of the microdevice containing human plasma (3,000-fold and 10,000-fold dilution) mixed with the fluorescent probe (compound 17; 30 µM) and acetylthiocholine iodide (1 mM) in HEPES buffer (10 mM, pH 7.4, containing 0.1% CHAPS). The image was acquired after incubation at 25°C for 30 minutes.

In a state where the blood was diluted to 1/10,000, enzyme activity at a single molecule level was detected, and λ = 0.03 (corresponding to a total enzyme concentration of 1 pM) was obtained. It was suggested that the enzyme activity was detected by separating the enzyme in the blood for each molecule.

### (2) Comparison of Fluorescence Intensities

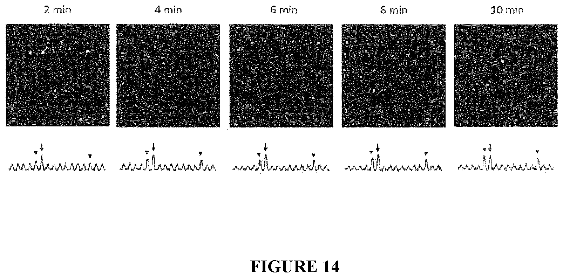

From the number of bright spots in the microdevice, it was found that the blood enzyme was separated for each molecule. The fluorescence intensity is also important for the evaluation of the enzyme activity. The enzyme in the blood was observed in a time course using the human blood sample. The results are illustrated in Fig. 14.

Fig. 14 illustrates time course analysis of a fluorescence image of a microdevice containing human plasma.

The upper view is a fluorescence image of a microdevice equipped with a probe (30 µM), acetylthiocholine (1 mM), and a 1/10,000-fold diluted plasma sample of a healthy human subject. The images were acquired 2 to 10 minutes after sealing. A white arrow indicates a chamber containing an enzyme with a high reaction rate, and a white arrowhead indicates a chamber containing an enzyme with a low reaction rate.

The lower view illustrates normalized fluorescence intensity of the device by line scan analysis along the ROI indicated by the white line in the image after 10 minutes.

At the stage of 2 min, there were bright spots with high fluorescence intensity and bright spots with low fluorescence intensity, and the reaction rate was significantly fast. Therefore, signals were saturated at about 10 min in both cases, and a result suggesting the completion of the reaction was obtained. From this, it was confirmed that there were enzymes reacting with acetylthiocholine with at least two different time constants in the blood. Since the influence of fading (about 5%/captured image) cannot be ignored in the case of time course imaging, hereinafter, the activity pattern was measured by setting 5 min as the reaction time during which signals can be stably acquired including the operation time and both signals can be separated.

### (3) Confirmation of Activity Pattern

Using the human blood sample, bright spot extraction was performed on GA3 analysis software of Nikon NIS for the acquired image, and the number of bright spots and the intensity of bright spots were analyzed.

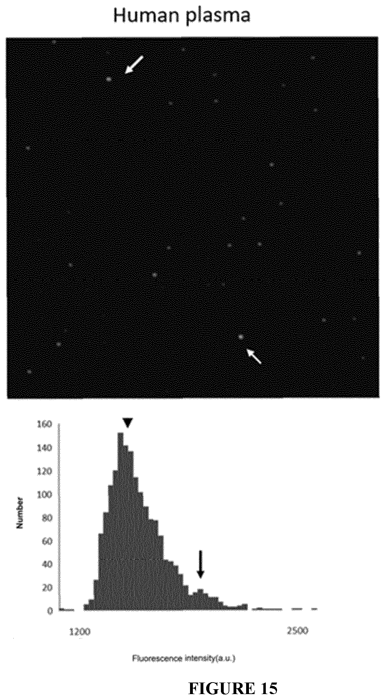

Fig. 15 illustrates the obtained fluorescence image of the microdevice containing human plasma.

The upper view is a fluorescence image of the microdevice containing human plasma (10,000-fold dilution) mixed with the fluorescent probe (compound 17; 30 µM) and acetylthiocholine iodide (1 mM) in HEPES buffer (10 mM, pH 7.4, containing 0.1% CHAPS). The image was acquired after incubation at 25°C for 5 minutes.

The lower view illustrates a histogram showing the number of chambers plotted and the increase in fluorescence.

When the bright spot of the blood sample was analyzed, a mountain (left arrow and arrow head in Fig. 15) having two large activity values was confirmed. From the small abundance ratio and the high reaction rate, it is expected that an enzyme having a high activity value corresponds to AChE.

### [Example 5]

### Detection Experiment of Glyoxalase 2 (GLO2)

GLO2 is responsible for one corner of a glyoxalase metabolic pathway responsible for a 2-methylglyoxal metabolic system, and it is known that activity abnormality occurs in many cancers. Since GLO2 metabolizes S-D-lactoyl-glutathione (see the following reaction formula), it is possible to construct a coupled assay using the probe of the present invention. 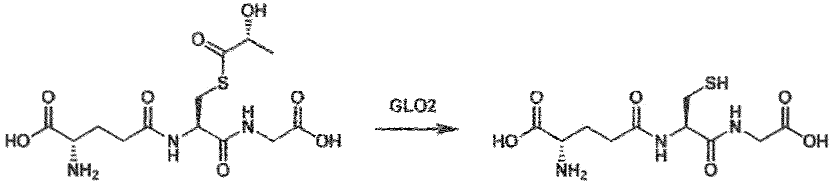 S-D-lactoyl-glutathione

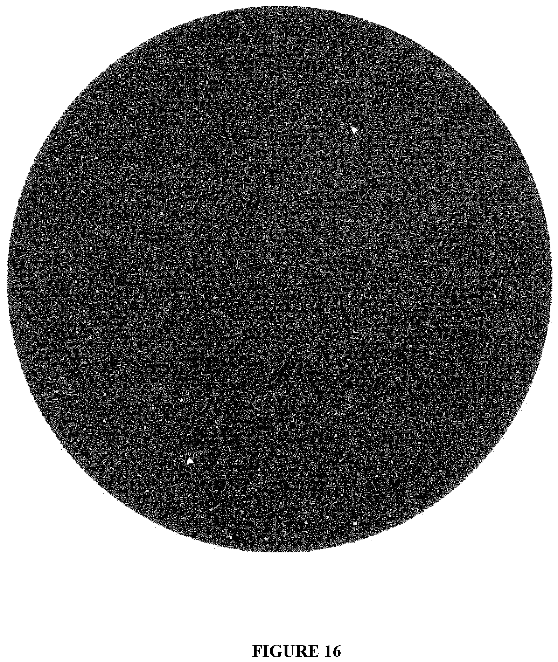

The results of detecting the activity of hydrolyzing an ester of glutathione (considered as GLO2) in blood are illustrated in Fig. 16.

Fig. 16 is a fluorescence image of the microdevice containing human plasma (1,000-fold dilution) mixed with the fluorescent probe (compound 17; 30 µM) and S-D-lactoyl-glutathione (1 mM) in HEPES buffer (10 mM, pH 7.4, containing 0.1% CHAPS). The image was acquired after incubation at 25°C overnight.

### [Example 6]

In the fluorescent probe of the present invention, in addition to the nitroolefin shown in Examples 1 to 4, a highly electrophilic compound such as nitrophenols or maleimide, or a group thereof can be used as a reaction point that reacts with the substrate analog. The results of verifying the applicability of nitrophenols to single-molecule measurement and the results of verifying the applicability of maleimides to a coupled assay are shown below.

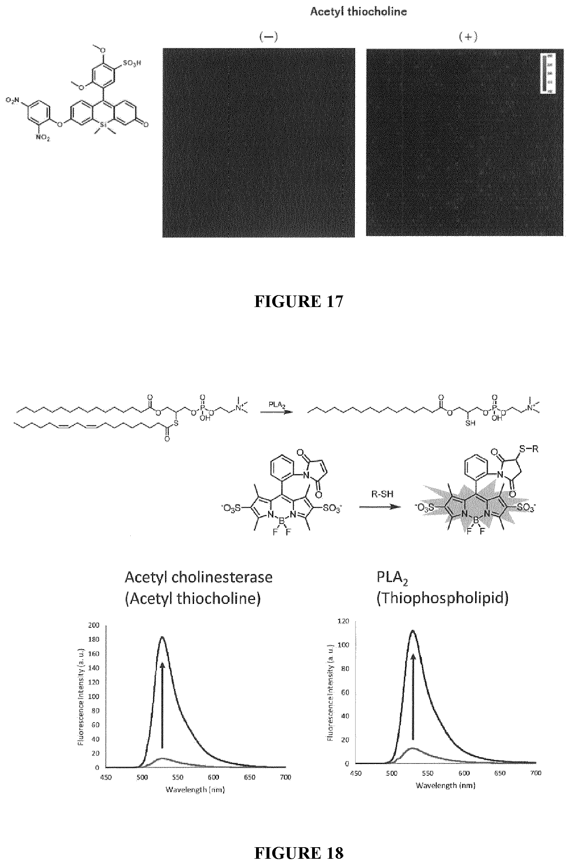

(1) Fig. 17 is a fluorescence image of the microdevice containing human plasma (1,000-fold dilution) mixed with the fluorescent probe (2.4-dinitro sTM, 1µM) of the present invention represented by the formula below and acetylthiocholine iodide (1 mM) in HEPES buffer (10 mM, pH 7.4, containing 0.1% CHAPS). The images were acquired after 90 minutes at 37°C. 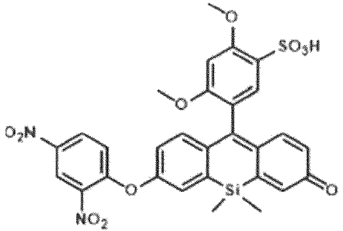 As illustrated in Fig. 17, the activity of acetylcholinesterase in blood was detected by using the probe of the present invention having a nitrophenol structure.
(2) Fig. 18 illustrates the activity detection results of AChE and phospholipase (PLA2) using a probe having a maleimide structure.

In the experiment, thiophospholipids represented by the formula below as P-substrate analogs were used in the coupled assay of LA2.

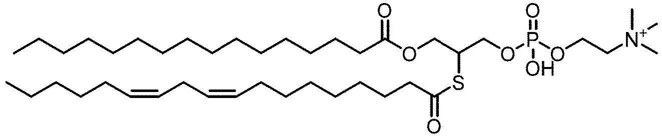

Fig. 18 illustrates the fluorescence spectra measured by adding a probe having a maleimide group shown in the drawing to a solution obtained by reacting the sulfur-containing lipid analog described above with phospholipase A2 (PLA2) An increase in fluorescence intensity was observed in the presence of PLA2, and a result suggesting that the activity of PLA2 was measured by the coupled assay according to the present invention was obtained.

## Claims

1. A method for detecting activity of an enzyme in a biological sample using a microdevice, the method comprising a step of bringing the biological sample into contact with a substrate analog or a natural substrate, and a compound represented by General Formula (I) below or a salt thereof,
wherein the substrate analog or the natural substrate generates a compound A having a thiol group (R-SH), a selenol group (R-SeH), or a poly sulfur atom (R-(S)n-H) (R is a hydrogen atom or an alkyl group, and n is 1 to 5) by a reaction with the enzyme, and
fluorescence is observed or measured, the fluorescence being emitted or enhanced from a fluorescent product generated by the reaction of the compound represented by General Formula (I) or the salt thereof with the thiol group, the selenol group, or the poly sulfur atom, 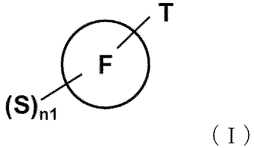
wherein
T is a reactive site that reacts with the thiol group (R-SH), the selenol group (R-SeH), or the poly sulfur atom (R-(S)n-H);
S is a hydrophilic group selected from the group consisting of a sulfonic acid group, a carboxyl group, and a phosphonic acid group;
n1 represents an integer of 1 to 10; 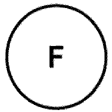
represents a fluorophore, in which molecular design is carried out so that optical properties of the entire molecule of the compound change by the reaction of the compound represented by General Formula (I) or the salt thereof with the thiol group, the selenol group, or the poly sulfur atom; and
S and T may be bonded to the fluorophore through a linker.

2. The method according to claim 1, wherein the substrate analog has a thioester- or selenoester-like structure and generates a corresponding thiol group or selenol group by an enzyme reaction.

3. The method according to claim 1, wherein the fluorophore is selected from the group consisting of a BODIPY-based fluorophore, a rhodamine-based fluorophore, a fluorescein-based fluorophore, a rhodol analog fluorophore, a cyanine-based fluorophore, a resorufin-based fluorophore, and a coumarin-based fluorophore.

4. The method according to claim 3, wherein the reactive site is selected from the group consisting of a nitroolefin group that may have a substituent, maleimides, an electron-deficient aryl group, and an alkyl or arylsulfonic acid group (R'-SO₃-; R' represents an alkyl group that may have a substituent or an aryl group that may have a substituent).

5. The method according to claim 2, wherein the substrate analog is one or more selected from the group consisting of acetylthiocholine, butylthiocholine, or a sulfur-containing lipid.

6. The method according to claim 1, wherein the enzyme to be detected is one or more selected from the group consisting of esterase, lipase, glyoxalase, SAH hydrolase, N-acetyltransferase, cystathionine γ-lyase, S-adenosylhomocysteine hydrolase, and cystathionine β-synthase.

7. The method according to claim 6, wherein the enzyme is acetylcholinesterase, phospholipase A2, or glyoxalase 2.

8. A fluorescent probe for use in a method for detecting activity of an enzyme in a biological sample using a microdevice, the method comprising a step of bringing the biological sample into contact with a substrate analog or a natural substrate, and a compound represented by General Formula (I) below or a salt thereof,
wherein the substrate analog or the natural substrate generates a compound A having a thiol group (R-SH), a selenol group (R-SeH), or a poly sulfur atom (R-(S)n-H) (R is a hydrogen atom or an alkyl group, and n is 1 to 5) by a reaction with the enzyme,
fluorescence is observed or measured, the fluorescence being emitted or enhanced from a fluorescent product generated by the reaction of the compound represented by General Formula (I) or the salt thereof with the thiol group, the selenol group, or the poly sulfur atom, and
the substrate analog generates a compound A having a thiol group, a selenol group (R-SeH), or a poly sulfur atom (R-(S)n-H) by a reaction with the enzyme,
the fluorescent probe comprising a compound represented by General Formula (I) or a salt thereof, 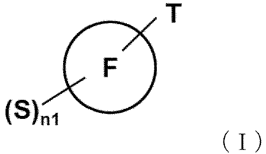
wherein
T is a reactive site that reacts with the thiol group (R-SH), the selenol group (R-SeH), or the poly sulfur atom (R-(S)n-H);
S is a hydrophilic group selected from the group consisting of a sulfonic acid group, a carboxyl group, and a phosphonic acid group;
n1 represents an integer of 1 to 10; 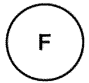
represents a fluorophore, in which molecular design is carried out so that optical properties of the entire molecule of the compound change by the reaction of the compound represented by General Formula (I) or the salt thereof with the thiol group, the selenol group, or the poly sulfur atom; and
S and T may be bonded to the fluorophore through a linker.

9. The fluorescent probe according to claim 8, wherein the compound represented by General Formula (I) or the salt thereof is a compound represented by General Formula (II) below or a salt thereof, the compound having one to five hydrophilic groups selected from the group consisting of a sulfonic acid group, a carboxyl group, and a phosphonic acid group, and having one to three reactive sites that react with the thiol group, the selenol group, or the poly sulfur atom, 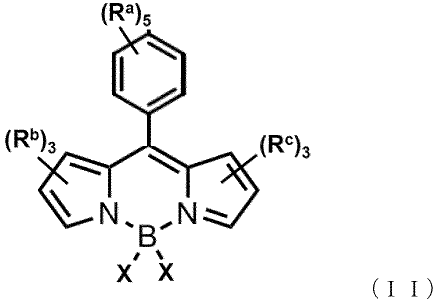 wherein
R^{a} is each independently a hydrogen atom, a monovalent substituent, the hydrophilic group, an alkyl group having the hydrophilic group and having 1 to 6 carbon atoms, or the reactive site;
R^{b} is each independently a hydrogen atom, a monovalent substituent, the hydrophilic group, an alkyl group having the hydrophilic group and having 1 to 6 carbon atoms, or the reactive site;
R^{c} is each independently a hydrogen atom, a monovalent substituent, the hydrophilic group, an alkyl group having the hydrophilic group and having 1 to 6 carbon atoms, or the reactive site; and
X is each independently selected from a fluorine atom, an alkyl group, an alkoxy group, an aryl group, the hydrophilic group, or an alkyl group having the hydrophilic group and having 1 to 6 carbon atoms.

10. The fluorescent probe according to claim 9, wherein the compound represented by General Formula (II) or the salt thereof is a compound represented by General Formula (IIa) below or a salt thereof, 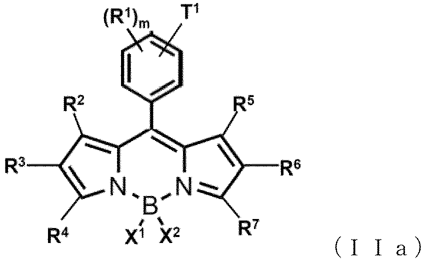 wherein
R¹, when present, represents the same or different monovalent substituent present on a benzene ring, and the substituent may have the hydrophilic group;
m is an integer of 0 to 4, and when m is 2 or more, each R¹ may be the same or different;
T¹ is a reactive site that reacts with the thiol group, the selenol group, or the poly sulfur atom, and two or more T¹s may be bonded to the benzene ring;
R² to R⁷ each independently represent a hydrogen atom, an alkyl group having 1 to 6 carbon atoms, the hydrophilic group, or an alkyl group having the hydrophilic group and having 1 to 6 carbon atoms; and
X¹ and X² are each independently selected from a fluorine atom, an alkyl group, an alkoxy group, an aryl group, the hydrophilic group, or an alkyl group having the hydrophilic group and having 1 to 6 carbon atoms.

11. The fluorescent probe according to claim 10, wherein the compound represented by General Formula (IIa) or the salt thereof is a compound represented by General Formula (IIb) below or a salt thereof, 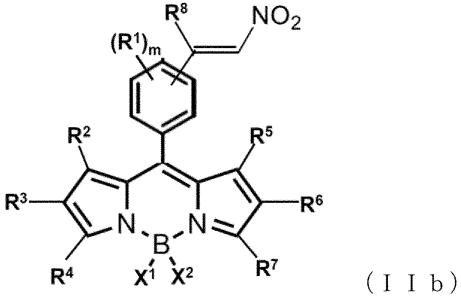 wherein
R¹ to R⁷, X¹, X², and m are as defined in General Formula (IIa); and
R⁸ represents a hydrogen atom or a substituted or unsubstituted alkyl group having 1 to 10 carbon atoms, and
two or more nitroolefin moieties that may have a substituent may be bonded to the benzene ring,
where at least one of R³ and R⁶ is the hydrophilic group.

12. The fluorescent probe according to claim 10, wherein the compound represented by General Formula (IIa) or the salt thereof is a compound represented by General Formula (IIc) below or a salt thereof, 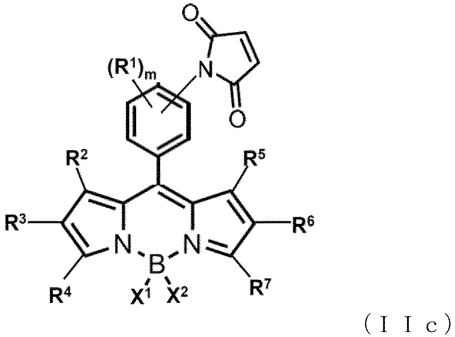 wherein
R¹ to R⁷, X¹, X², and m are as defined in General Formula (IIa); and
two or more maleimide groups may be bonded to the benzene ring,
where at least one of R³ and R⁶ is the hydrophilic group.

13. The fluorescent probe according to claim 8, wherein the compound represented by General Formula (I) or the salt thereof is a compound represented by General Formula (III) below or a salt thereof, the compound having one to five hydrophilic groups selected from the group consisting of a sulfonic acid group, a carboxyl group, and a phosphonic acid group, and having one to three reactive sites that react with the thiol group, the selenol group, or the poly sulfur atom, 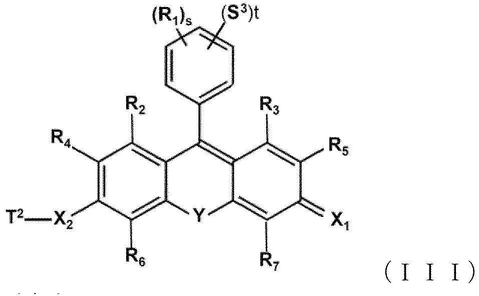 wherein
R₁, when present, represents the same or different monovalent substituent present on a benzene ring;
s is an integer of 0 to 4, and when s is 2 or more, each R₁ may be the same or different;
S³ is a hydrophilic group selected from a sulfonic acid group, a carboxyl group, or a phosphonic acid group;
t is 0 to 5;
R₂ to R₇ each independently represent a hydrogen atom, an alkyl group having 1 to 6 carbon atoms, a halogen atom, the hydrophilic group, or an alkyl group having the hydrophilic group and having 1 to 6 carbon atoms;
T² is a reactive site that reacts with the thiol group, the selenol group, or the poly sulfur atom;
X₁ and X₂ are selected from combinations of (O,O), (O,NRs), (NR₉R₁₀,NR₈), and NR₉R₁₀,NR₈), where R₈ to R₁₀ are each independently a hydrogen atom, a substituted or unsubstituted alkyl group having 1 to 6 carbon atoms, a substituted or unsubstituted alkenyl group having 1 to 6 carbon atoms, an alkyl group having the hydrophilic group and having 1 to 6 carbon atoms, or an alkenyl group having the hydrophilic group and having 1 to 6 carbon atoms; and
Y is selected from an oxygen atom, Si(Rₐ)(R_{b}), C(Rₐ)(R_{b}), P(=O)R_{c}, S(R_{d})₂, Ge(Rₐ)(R_{b}), a tellurium atom, or a bismuth atom,
where Rₐ and R_{b} are each independently a substituted or unsubstituted alkyl group having 1 to 6 carbon atoms or a substituted or unsubstituted aryl group,
R_{c} is a substituted or unsubstituted alkyl group having 1 to 6 carbon atoms or a substituted or unsubstituted phenyl group,
R_{d} is the same or different substituted or unsubstituted aryl group, and
the alkyl group, the aryl group, and the phenyl group of Rₐ to R_{d} may be substituted with the hydrophilic group.

14. The fluorescent probe according to claim 13, wherein the reactive site of T² is any of the following: 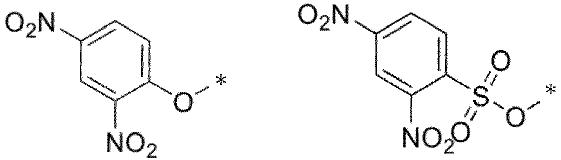 wherein * represents a binding site.

15. The fluorescent probe according to claim 8, wherein the substrate analog has a thioester- or selenoester-like structure and generates a corresponding thiol group or selenol group by an enzyme reaction.

16. The fluorescent probe according to claim 15, wherein the substrate analog is one or more selected from the group consisting of acetylthiocholine, butylthiocholine, and a sulfur-containing lipid derivative.

17. The fluorescent probe according to claim 8, wherein the enzyme to be detected is one or more selected from the group consisting of esterase, lipase, glyoxalase, SAH hydrolase, N-acetyltransferase, cystathionine γ-lyase, S-adenosylhomocysteine hydrolase, and cystathionine β-synthase.

18. The fluorescent probe according to claim 17, wherein the enzyme is acetylcholinesterase, phospholipase A2, or glyoxalase 2.

19. The fluorescent probe according to claim 18, which is used to detect activity of acetylcholinesterase in a biological sample including blood.

20. A compound represented by General Formula (IIb) below or a salt thereof, 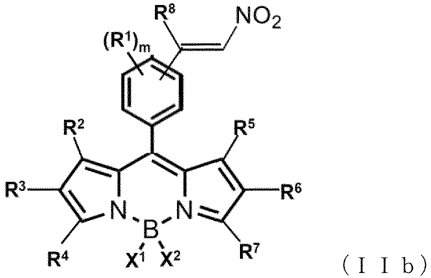 wherein
R¹, when present, represents the same or different monovalent substituent present on a benzene ring, and the substituent may have a hydrophilic group selected from the group consisting of a sulfonic acid group, a carboxyl group, and a phosphonic acid group;
m is an integer of 0 to 4, and when m is 2 or more, each R¹ may be the same or different;
R² to R⁷ each independently represent a hydrogen atom, an alkyl group having 1 to 6 carbon atoms, the hydrophilic group, or an alkyl group having the hydrophilic group and having 1 to 6 carbon atoms;
R⁸ represents a hydrogen atom or a substituted or unsubstituted alkyl group having 1 to 10 carbon atoms,
provided that at least one of R³ and R⁶ is a sulfonic acid group, a carboxyl group, or a phosphonic acid group; and
X¹ and X² are each independently selected from a fluorine atom, an alkyl group, an alkoxy group, an aryl group, the hydrophilic group, or an alkyl group having the hydrophilic group and having 1 to 6 carbon atoms.

21. The compound or a salt thereof according to claim 20, wherein at least one of R³ and R⁶ is a sulfonic acid group.

22. The compound or a salt thereof according to claim 20, wherein at least one of R³ and R⁶ is a carboxyl group.

23. The compound or a salt thereof according to claim 20, wherein at least one of R³ and R⁶ is a phosphonic acid group.

24. The compound or a salt thereof according to claim 20, wherein m is 1 to 2, and R¹ is an alkoxyl group having 1 to 6 carbon atoms.

25. The compound or a salt thereof according to claim 20, wherein a nitroolefin moiety is attached to the 2- or 4-position of the benzene ring.

26. The compound or a salt thereof according to claim 22, wherein R³ to R⁶ each represent a methyl group.

27. A compound selected from the following or a salt thereof
